(19) Europäisches Patentamt
European Patent Office
Office européen des brevets

(11) Veröffentlichungsnummer: **0 300 278**
**A2**

(12) # EUROPÄISCHE PATENTANMELDUNG

(21) Anmeldenummer: **88110846.8**

(22) Anmeldetag: **07.07.88**

(51) Int. Cl.⁴: **C07D 207/337 , C07D 207/333 , C07D 405/06**

(30) Priorität: **21.07.87 DE 3724112**
**26.05.88 DE 3817808**

(43) Veröffentlichungstag der Anmeldung:
**25.01.89 Patentblatt 89/04**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Hübsch, Walter, Dr.**
**Am Eckbusch 43/50**
**D-5600 Wuppertal 1(DE)**
Erfinder: **Angerbauer, Rolf, Dr.**
**Sterntalerweg 29**
**D-5600 Wuppertal 1(DE)**
Erfinder: **Fey, Peter, Dr.**
**Ursulastrasse 14**
**D-5600 Wuppertal 1(DE)**
Erfinder: **Bischoff, Hilmar, Dr.**
**Am Rohm 78**
**D-5600 Wuppertal 2(DE)**
Erfinder: **Petzinna, Dieter, Dr.**
**Dr.-Peter-Berten-Strasse 10**
**D-4000 Duesseldorf 12(DE)**
Erfinder: **Schmidt, Delf, Dr.**
**Am Eckbusch 55 b**
**D-5600 Wuppertal 1(DE)**

(54) **Verfahren zur Herstellung von 2-substituierten Pyrrolen.**

(57) 2-Substituierte Pyrrole werden durch Reduktion von entsprechenden Ketonen und gegebenenfalls nachfolgender Verseifung, Cyclisierung, Hydrierung und Isomerentrennung hergestellt. Die Ketone sind neu und können aus entsprechenden, ebenfalls neuen, Aldehyden durch Umsetzung mit einem Acetessigester hergestellt werden. Die Aldehyde können durch Umsetzung von Pyrrolen mit N,N-Dialkylacrolein hergestellt werden.

EP 0 300 278 A2

## Verfahren zur Herstellung von 2-substituierten Pyrrolen

Die Erfindung betrifft ein Verfahren zur Herstellung von 2-substituierten Pyrrolen und Zwischenverbindungen zu ihrer Herstellung.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [EP-A 22 478; US 4 231 938]. Darüberhinaus sind auch bestimmte Indolderivate bzw. Pyrazolderivate Inhibitoren der HMG-CoA-Reduktase [EP-A 1 114 027; US-Patent 4 613 610].

Aus der EP-A 0 221 025 sind 2-substituierte Pyrrole bekannt. Ihre Herstellung ist umständlich und erfolgt über viele Zwischenstufen.

Es wurde ein Verfahren zur Herstellung von 2-substituierten Pyrrolen der allgemeinen Formel (I)

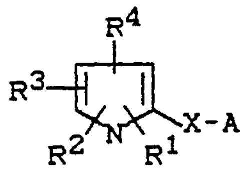

(I)

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl, oder durch eine Gruppe der Formel $-NR^5R^6$,

worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$ und $R^6$ die oben angegebene Bedeutung haben,

oder

- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

$R^3$ und $R^4$ gleich oder verschieden sind und

- für Wasserstoff oder

- für Acyl, Alkylsulfonyl oder Arylsulfonyl stehen, wobei der Arylrest durch Alkyl oder Halogen substituiert sein kann, oder

- für Aminocarbonyl, Alkylaminocarbonyl, Dialkylaminocarbonyl, Arylaminocarbonyl oder Alkoxycarbonyl stehen, oder

- für Cycloalkyl stehen, oder

- für Alkyl stehen, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio,

Arylsulfonyl, Aralkoxy, Aralkylthio oder Aralkylsulfonyl, wobei die Heteroaryl- oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein können, worin
$R^5$ und $R^6$ die oben angegebene Bedeutung haben,
oder
- für Aryl stehen, die bis zu 5-fach gleich oder verschieden substituiert sein können durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben X - für eine Gruppe der Formel $-CH_2-CH_2$ oder $-CH=CH-$steht,
und
A - für eine Gruppe der Formel

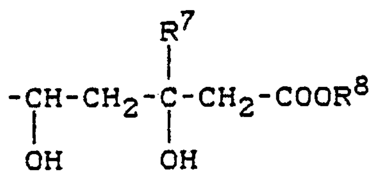 oder 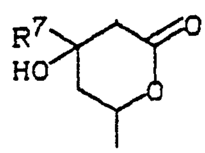 steht,

worin
$R^7$ - Wasserstoff oder Alkyl bedeutet
und
$R^8$ - Wasserstoff,
- einen physiologisch verträglichen Esterrest oder
- ein physiologisch verträgliches Kation bedeutet,
gefunden,
das dadurch gekennzeichnet ist, daß man Ketone der allgemeinen Formel (VIII)

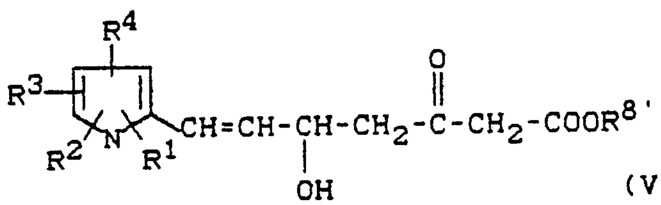
(VIII)

in welcher
$R^1$, $R^2$, $R^3$ und $R^4$ die oben angegebene Bedeutung haben,
und
$R^8$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen ($X = -CH_2-CH_2-$) die Ethenverbindungen ($X = -CH=CH-$) nach üblichen Methoden hydriert,
und gegebenenfalls Isomeren trennt.

Das erfindungsgemäße Verfahren kann durch das folgendes Formelschema erläutert werden:

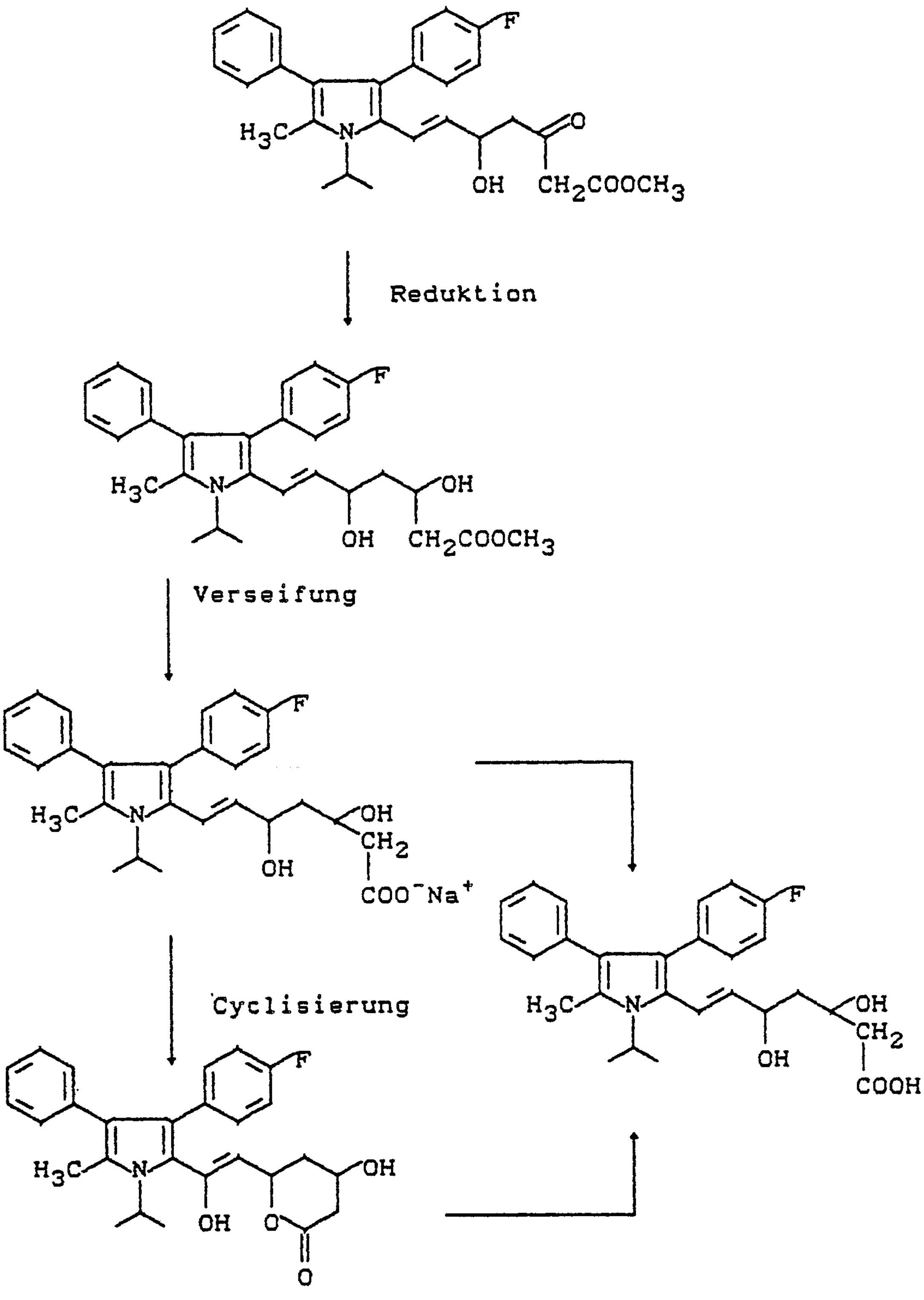


Im Rahmen der vorliegenden Erfindung können die Substituenten im allgemeinen die folgende Bedeutung haben:

Cycloalkyl steht im allgemeinen für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder

verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethylsulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl im Rahmen der oben angebenen Definition steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoaxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Steht $R^8$ für einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_4$) und Aralkylester ($C_7$ bis $C_{10}$), bevorzugt Niederalkylester sowie Benzylester. Darüber hinaus seien die folgenden Esterreste genannt: Methylester, Ethylester, Propylester, Benzylester.

Steht $R^8$ für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniederalkylamine ($C_9$ bis etwa $C_6$), Triniederalkylamine ($C_1$ bis etwa $C_6$), Prokain, Dibenzylamin, N,N′-Dibenzylethylendiamin, N-Benzyl-β-phenylethylamin, N-Methylmorpholin oder N-Ethylmorpholin, 1-Ephenamin, Dihydroabietylamin, N,N′-Bis-dihydroabietylethylendiamin, N-Niederalkylpiperidin und andere Amine, die zur Bildung von Salzen verwendet werden können.

Im Rahmen der vorliegenden Erfindung entsprechen die 2-substituierten Pyrrole (Ia) der allgemeinen Formel

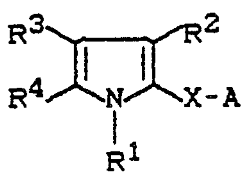

(Ia)

in welcher

$R^1$, $R^2$, $R^3$, $R^4$, X und A die oben angegebene Bedeutung haben.

Im Rahmen der vorliegenden Erfindung entsprechen die 2-substituierten Pyrrole (Ib) der allgemeinen Formel

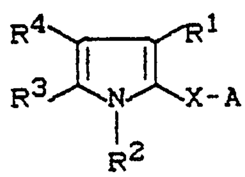

(Ib)

in welcher

$R^1$, $R^2$, $R^3$, $R^4$, X und A die oben angegebene Bedeutung haben.

Im Rahmen der allgemeinen Formel (I) sind Verbindungen mit dem allgemeinen Formeln (Ia) und (Ib) bevorzugt.

Bevorzugt sind solche Verbindungen der allgemeinen Formeln (Ia) und (Ib)

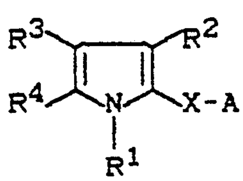

(Ia) oder

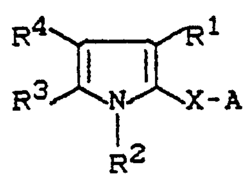

(Ib)

in welchen

$R^1$ - für Cyclopropyl, Cyclopentyl oder Cyclohexyl steht, oder

- für Niederalkyl steht, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,

worin

$R^5$ und $R^6$ gleich oder verschieden sind und Niederalkyl, Phenyl, Benzyl, Acetyl, Benzoyl, Phenylsulfonyl

oder Niederalkylsulfonyl bedeuten,

oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenyl ethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

$R^2$ - für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl steht, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- für Phenyl oder Naphthyl steht, die bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,

wobei

$R^5$ und $R^6$ die oben angegebene Bedeutung haben,

$R^3$ und $R^4$ gleich oder verschieden sind und

- für Wasserstoff oder
- für Benzoyl oder Niederalkylcarbonyl stehen, oder
- für Niederalkylsulfonyl,Phenylsulfonyl oder Tolylsulfonyl stehen, oder
- für Aminocarbonyl, Niederalkylaminocarbonyl, Diniederalkylaminocarbonyl, Phenylaminocarbonyl oder Niederalkoxycarbonyl stehen, oder
- für Cyclopropyl, Cyclopentyl oder Cyclohexyl stehen oder
- für Niederalkyl stehen, die substituiert sein können durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$, worin

$R^5$ und $R^6$ gleich oder verschieden sind und Niederalkyl, Phenyl, Benzyl, Acetyl, Benzoyl, Phenylsulfonyl oder Niederalkylsulfonyl bedeuten,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

- für Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl stehen, die bis zu 2-fach gleich oder verschieden substituiert sein können durch Fluor, Chlor, Brom, Niederalkyl, Niederalkoxy, Phenyl, Phenoxy, Trifluormethyl, Trifluormethoxy oder Niederalkoxycarbonyl, oder
- für Phenyl oder Naphthyl stehen, die bis zu 4-fach gleich oder verschieden substituiert sein können durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,

wobei

$R^5$ und $R^6$ die oben angegebene Bedeutung haben

X - für eine Gruppe der Formel $-CH_2-CH_2-$ oder $-CH=CH-$steht,

A - für eine Gruppe der Formel

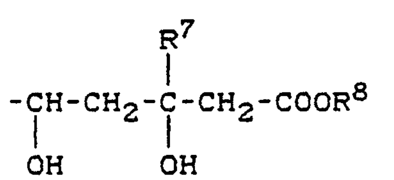 oder 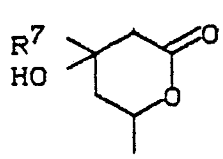 steht,

worin

$R^7$ - Wasserstoff oder Niederalkyl bedeutet,
und
$R^8$ - einen physiologisch verträglichen Esterrest bedeutet, oder
- ein physiologisch verträgliches Kation bedeutet.

Besonders bevorzugt sind Verbindungen der allgemeinen Formeln (Ia) und (Ib) ,
in welchen
$R^1$ - für Cyclopropyl, Cyclopentyl oder Cyclohexyl steht, oder
- für Methyl, Ethyl, Propyl, Isopropyl, Butyl, sec.-Butyl oder tert.Butyl steht, die substituiert sein können durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzoylthio oder Benzylsulfonyl,
$R^2$ - für Pyridyl, Pyrimidyl, Chinolyl oder Isochinolyl stehen, die durch Fluor, Chlor, Methyl, Methoxy oder Trifluormethyl substituiert sein können, oder
- für Phenyl stehen, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
$R^3$ und $R^4$ gleich oder verschieden sind und
- für Wasserstoff, Cyclopropyl, Cyclopentyl oder Cyclohexyl stehen, oder
- für Methyl, Ethyl, Propyl, Isopropyl, Butyl, Isobutyl, tert.Butyl, Pentyl, Isopentyl, Hexyl oder Isohexyl stehen, die substituiert sein können durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^5R^6$,
wobei
$R^5$ und $R^6$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- für Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl stehen, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- für Phenyl stehen, die bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^5R^6$ substituiert sein können,
wobei
$R^5$ und $R^6$ die oben angegebene Bedeutung haben,
oder
- für Benzoyl, Acetyl, Ethylcarbonyl, Propylcarbonyl, Isopropylcarbonyl, Methylsulfonyl, Ethylsulfonyl, Propylsulfonyl, Isopropylsulfonyl, Butylsulfonyl, Isobutylsulfonyl, tert.Butylsulfonyl, Phenylsulfonyl oder Tolylsulfonyl stehen, oder

- für Aminocarbonyl, Methyl-, Ethyl-, Propyl-, Isopropyl-, Butyl-, Isobutyl- oder tert.Butylaminocarbonyl, Dimethyl-, Diethyl-, Dipropyl-, Diisopropyl-, Dibutyl- oder Diisobutylaminocarbonyl, Phenylaminocarbonyl,Methoxycarbonyl, Ethoxycarbonyl, Propoxycarbonyl, Isopropoxycarbonyl, Butoxycarbonyl, Isobutoxycarbonyl oder tert.Butoxycarbonyl stehen,

X - für eine Gruppe der Formel $-CH_2-CH_2-$ oder -CH=CH-steht,

A - für eine Gruppe der Formel

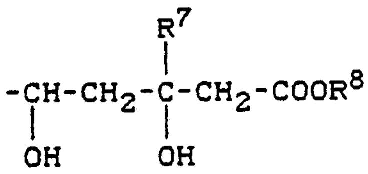 oder 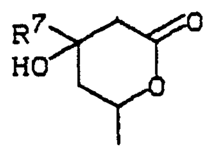 steht,

worin

$R^7$ - Wasserstoff, Methyl, Ethyl, Propyl, Isopropyl, Butyl, Isobutyl oder tert.Butyl bedeutet,

und

$R^8$ - Wasserstoff, Methyl, Ethyl, Propyl, Isopropyl, Butyl, Isobutyl, tert.Butyl oder Benzyl bedeutet, oder ein Natrium-, Kalium-, Calcium -, oder Magnesium- oder Ammoniumion bedeutet.

Die nach dem erfindungsgemäßen Verfahren hergestellten 2-substituierten Pyrrole der allgemeinen Formel (I) haben mehrere asymmetrische Kohlenstoffatome und können daher in verschiedenen stereochemischen Formen existieren.

Je nach der Bedeutung der Gruppe X bzw. des Restes A ergeben sich unterschiedliche Stereoisomere, die im folgenden näher erläutert werden sollten:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

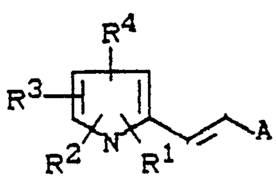

(II) E-Form

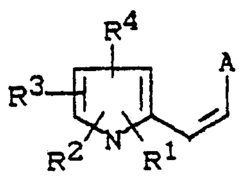

(III) Z-Form

($R^1$ bis $R^4$, A haben die oben angegebene Bedeutung).

Bevorzugt sind diejenigen Verbindungen der allgemeinen Formel (I) die E-konfiguriert sind (II).

b) Steht der Rest -A- für eine Gruppe der Formel

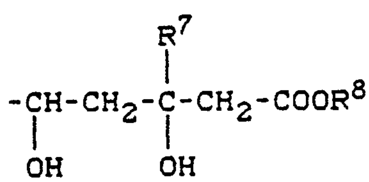

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

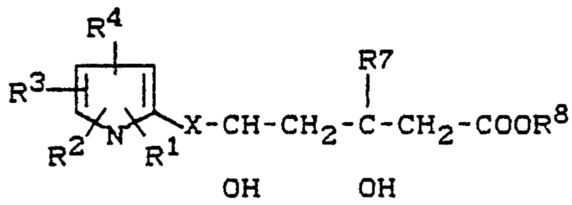

Erythro-Form (IV)

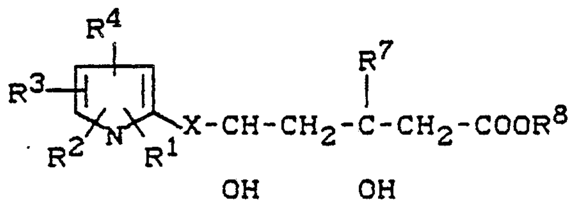

Threo-Form (V)

Sowohl von den Verbindungen in Erythro- als auch in Threo-Konfiguration existieren wiederum jeweils zwei Enantiomere, nämlich 3R,5S-Isomeres bzw. 3S,5R-Isomeres (Erythro-Form) sowie 3R,5R-Isomers und 3S,5S-Isomeres (Threo-Form).

Bevorzugt sind hierbei die Erythro-konfigurierten Isomeren, besonders bevorzugt das 3R,5S-Isomere sowie das 3R,5S-3S,5R-Racemat.

c) Steht der Rest -A- für eine Gruppe der Formel

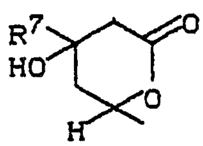

so besitzen die substituierten Pyrrole mindestens zwei asymmetrische Kohlenstoffatome, nämlich das Kohlenstoffatom an dem die Hydroxygruppe gebunden ist, und das Kohlenstoffatom an welchem der Rest der Formel

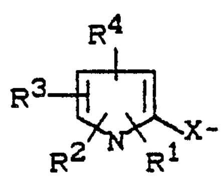

gebunden ist. Je nach der Stellung der Hydroxygruppe zur freien Valenz am Lactonring können die substituierten Pyrrole als cis-Lactone (VI) oder als trans-Lactone (VII) vorliegen.

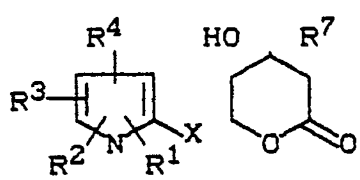

cis-Lacton (VI)

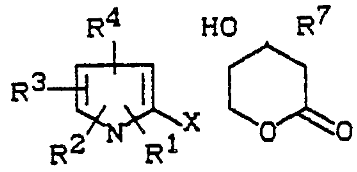

trans-Lacton (VII)

Sowohl vom cis-Lacton aus als auch vom trans-Lacton existieren wiederum jeweils zwei Isomeren nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrrole genannt:

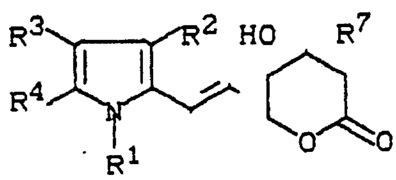

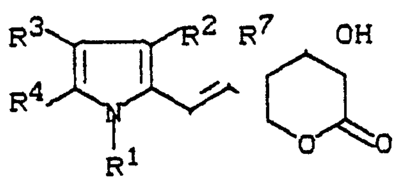

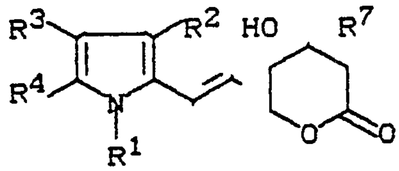

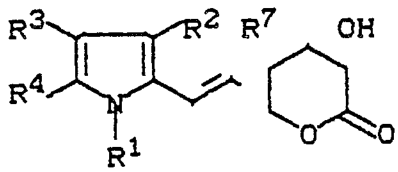

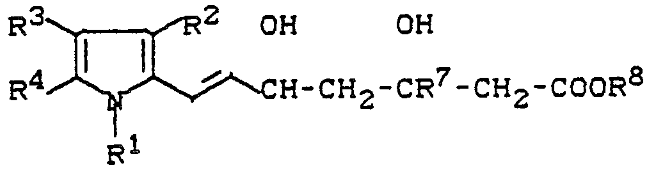

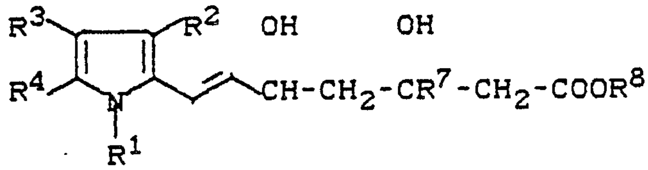

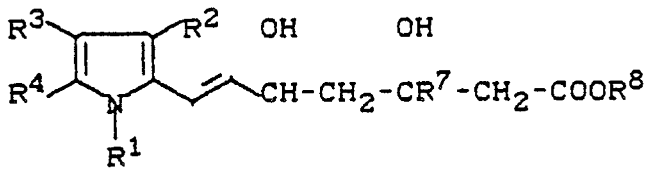

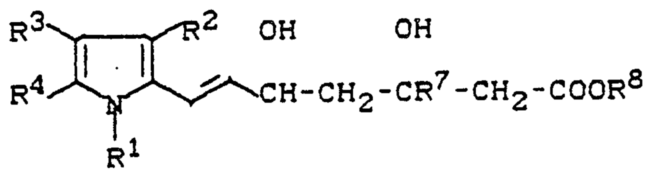

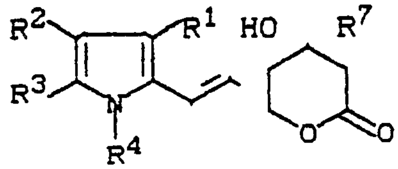

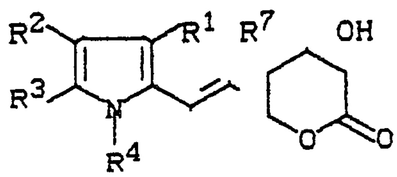

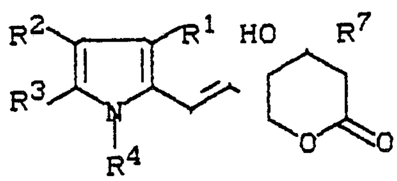

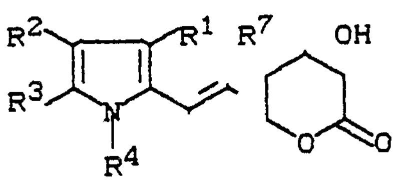

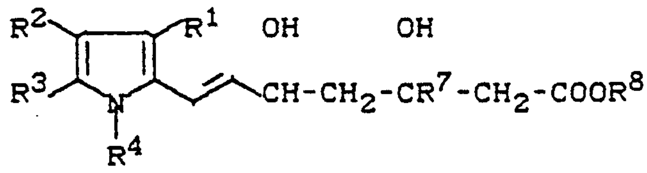

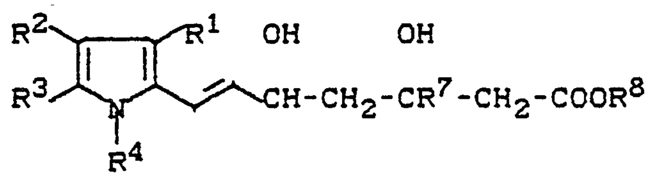

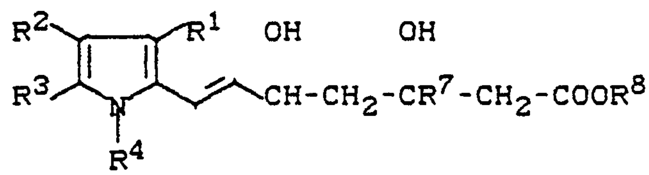

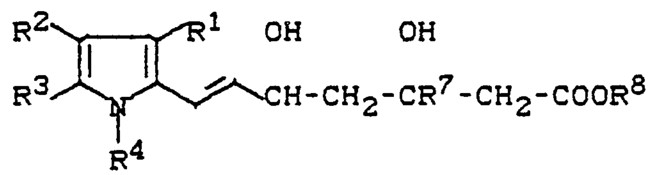

Ganz besonders bevorzugt sind die Verbindungen der allgemeinen Formel (Ia) und (Ib), in welchen
$R^1$ - für Cyclopropyl, Methyl, Ethyl, Propyl, Isopropyl, Butyl, Isobutyl oder tert.Butyl steht,
$R^2$ - für Phenyl steht, das bis zu 2-fach gleich oder verschieden durch Methyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Brom, Cyano, Trifluormethyl oder Trifluormethoxy substituiert sein kann,
$R^3$ und $R^4$ gleich oder verschieden sind und
- für Wasserstoff, Cyclopropyl, Cyclopentyl oder Cyclohexyl stehen, oder
- für Methyl, Ethyl, Propyl, Isopropyl, Butyl, Isobutyl, tert.Butyl, Pentyl, Isopentyl, Hexyl oder Isohexyl stehen,
oder
- für Phenyl stehen, die bis zu 2-fach gleich oder verschieden durch Methyl, Ethyl, Isopropyl, tert.Butyl, Methoxy, Ethoxy, Isopropoxy, Phenoxy, Fluor, Chlor, substituiert sein können,
X - für eine Gruppe der Formel
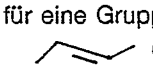 (E-konfiguriert) steht
und
A - für eine Gruppe der Formel

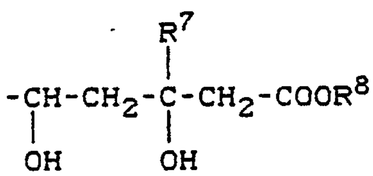 oder 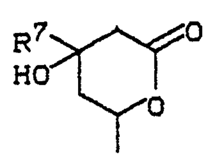 steht,

worin
$R^7$ - Wasserstoff bedeutet
und
$R^8$ - Wasserstoff, Methyl oder Ethyl bedeutet oder ein Natrium- oder Kaliumkation bedeutet.

Das erfindungsgemäße Verfahren zur Herstellung von 2-substituierten Pyrrolen wird im allgemeinen wie

folgt durchgeführt:

Die Reduktion kann mit üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden bevorzugt komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithiumtrialkyl-hydrido-boranaten, Natrium-trialkyl-hydridoboranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel für das erfindungsgemäße Verfahren eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80° bis Raumtemperatur (etwa +30° C), bevorzugt von -78° C bis 0° C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (III) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

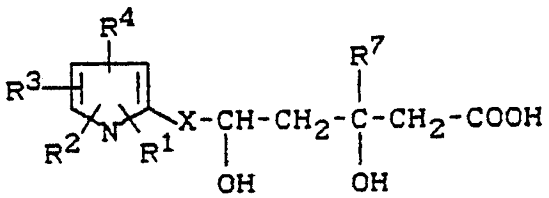

(Ic)

in welcher

$R^1$, $R^2$, $R^3$, $R^4$, $R^7$ und X die oben angegebene Bedeutung haben.

Die Carbonsäureester im Rahmen der allgemeinen Formel (I) entsprechen der Formel (Id)

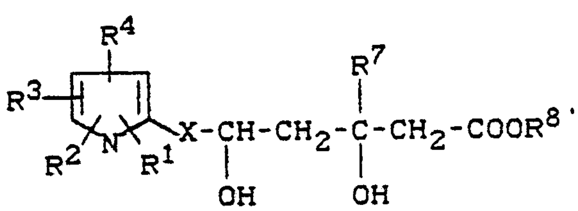

(Id)

in welcher

$R^1$, $R^2$, $R^3$, $R^4$, $R^7$ und X die oben angegebene Bedeutung haben,

und

$R^{8'}$ - für Alkyl steht.

Die Salze der erfindungsgemäßen Verbindungen im Rahmen der allgemeinen Formel (I) entsprechen der Formel (Ie)

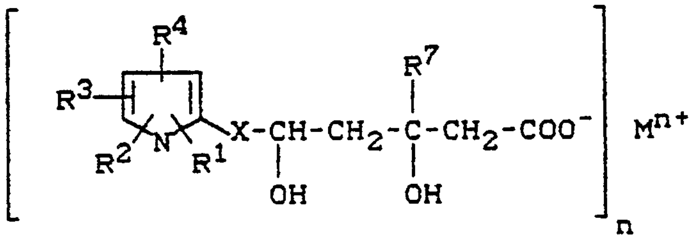

(Ie)

in welcher

$R^1$, $R^2$, $R^3$, $R^4$, $R^7$ und X die oben angegebene Bedeutung haben,

und

$M^n$ für ein Kation mit der Wertigkeit n steht.

Die bevorzugte Wertigkeit ist 1 oder 2.

Die Lactone im Rahmen der allgemeinen Formel (I) entsprechen der Formel (If)

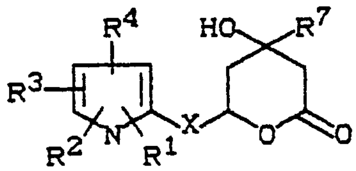

(If)

in welcher

$R^1$, $R^2$, $R^3$, $R^4$, $R^7$ und X die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäuern.

Die Säuren können dann in üblicher Weise isoliert werden.

Zur Hestellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40 °C bis +200 °C, bevorzugt von -25 °C bis +50 °C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cylisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbondiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0 °C bis +80 °C, bevorzugt von +10 °C bis +50 °C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

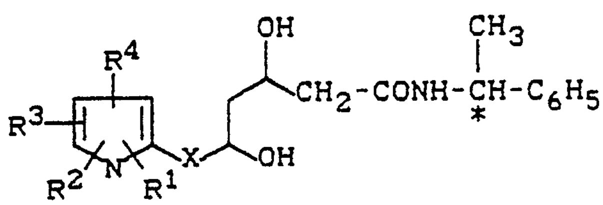

(Ig)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukt nach der oben beschriebenen Methode abhängig ist von der Konfiguration der Ausgangsstoffe.

Die Isomerentrennung soll im folgenden Schema beispielhaft erläutert werden:

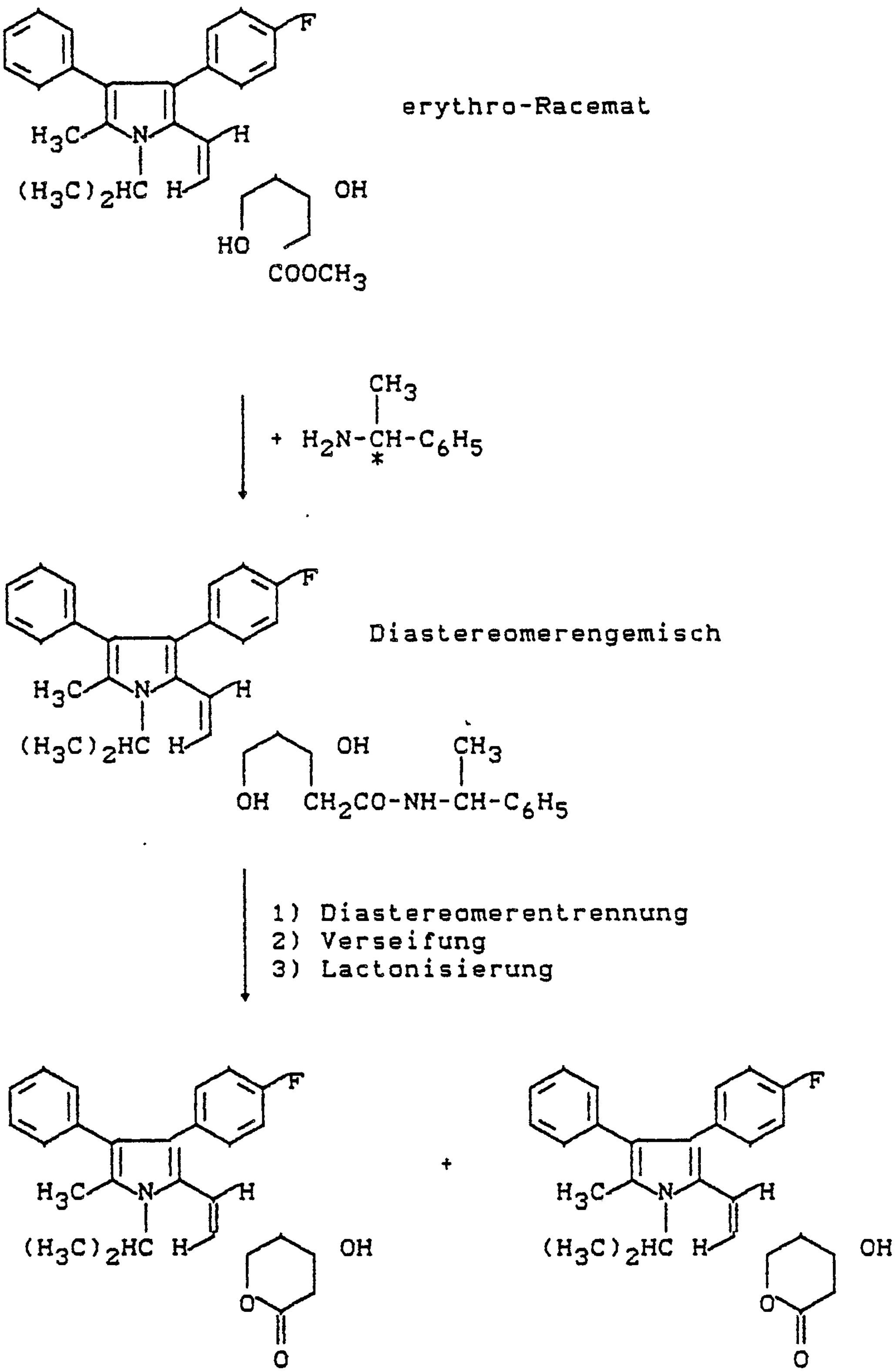


Die als Ausgangsstoffe für das erfindungsgemäße Verfahren eingesetzten Ketone (VIII) sind neu.

Es wurde ein Verfahren zur Herstellung der erfindungsgemäßen Ketone der allgemeinen Formel (VIII)

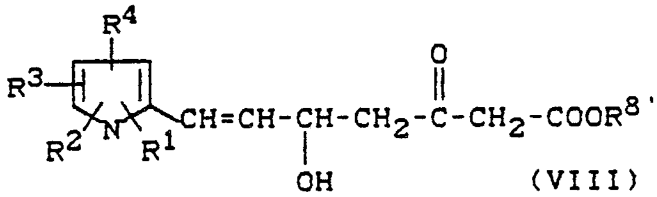

(VIII)

in welcher

$R^1$, $R^2$, $R^3$, $R^4$ und $R^8$ die oben angegebene Bedeutung haben,

gefunden, das dadurch gekennzeichnet ist, daß man Aldehyde der allgemeinen Formel (IX)

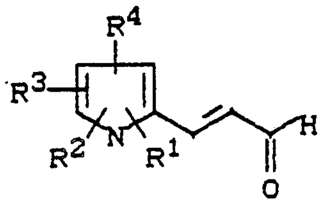

(IX)

in welcher

$R^1$, $R^2$, $R^3$ und $R^4$ die oben angegebene Bedeutung haben,

in inerten Lösemitteln mit einem Acetessigester der allgemeinen Formel (X)

$H_3C-\overset{O}{\overset{\|}{C}}-CH_2-COOR^8$ (X)

in welcher

$R^8$ die oben angegebene Bedeutung hat,

in Anwesenheit von Basen umsetzt.

Das erfindungsgemäße Verfahren zur Herstellung der Ketone kann beispielsweise durch folgendes Formelschema erläutert werden:

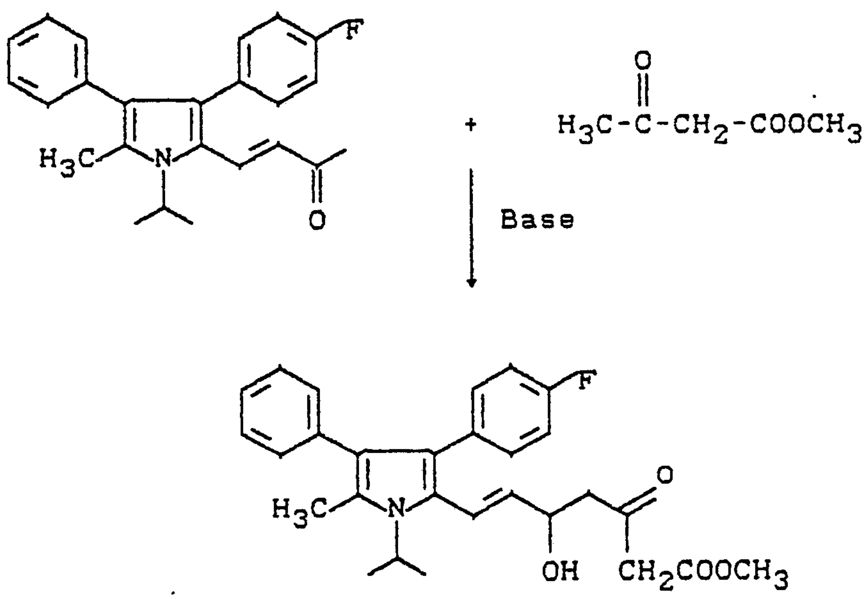


Als Basen kommen hierbei die stark basischen Verbindungen in Frage. Hierzu gehören bevorzugt lithiumorganische Verbindungen wie beispielsweise N-Butyllithium, sec.Butyllithium, tert.Butyllithium oder Phenyllithium, oder Amide wie beispielsweise Lithiumdiisopropyl amid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden N-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis Raumtemperatur (etwa +30 °C) durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von etwa 1 bis 2, bevorzugt von 1 bis 1,5 Mol, bezogen auf 1 Mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt: Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

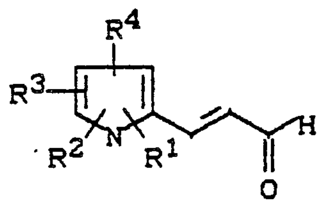

(IX)

in welcher
$R^1$, $R^2$, $R^3$ und $R^4$ die oben angegebene Bedeutung haben, gefunden, das dadurch gekennzeichnet ist, daß man Pyrrole der allgemeinen Formel (XI)

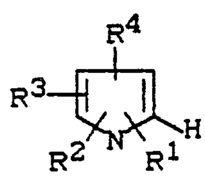

(XI)

in welcher
$R^1$, $R^2$, $R^3$ und $R^4$ die oben angegebene Bedeutung haben,
in inerten Lösemitteln in Anwesenheit von Säurechloriden mit N,N-Dialkylaminoacrolein der Formel (XII)

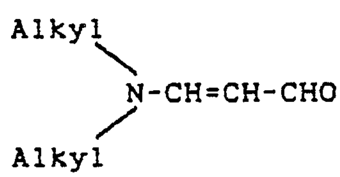

(XII)

wobei
Alkyl für einen geradkettigen oder verzweigten Kohlenwasserstoffrest mit 1 bis 6 Kohlenstoffatomen steht, umsetzt.

Das erfindungsgemäße Verfahren kann beispielsweise durch folgendes Formelschema verdeutlicht werden:

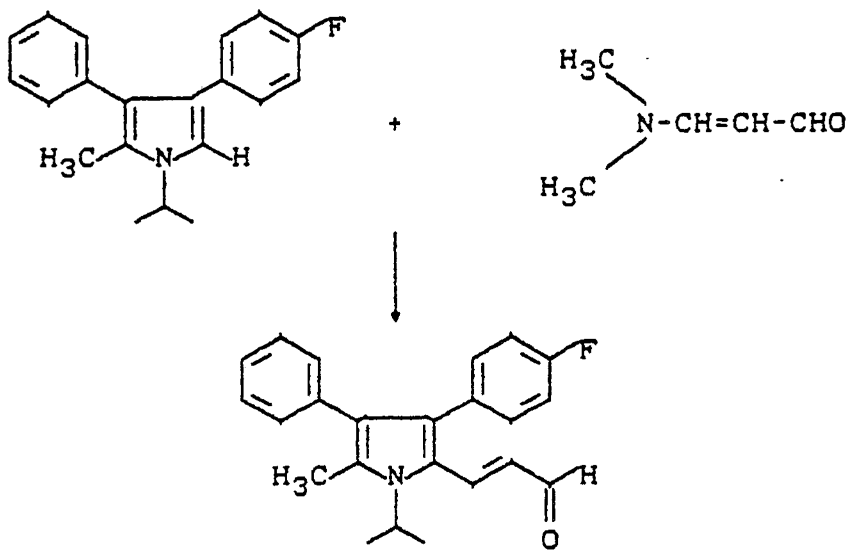

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff, oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20 °C bis +150 °C, bevorzugt von 0 °C bis +100 °C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgemeinen in einer Menge von 1 bis 6, bevorzugt von 1,2 bis 3 mol, bezogen auf 1 mol des Pyrrols eingesetzt.

Die als Ausgangsstoffe eingesetzten Pyrrole der allgemeinen Formel (XI) sind bekannt oder können nach bekannten Methoden hergestellt werden [A. Gossauer "Die Chemie der Pyrrole", Springer Verlag Berlin, 1974] (wie im experimentellen Teil beschrieben).

Es wurde außerdem ein verbessertes Verfahren zur Herstellung der Pyrrole der allgemeinen Formel (XI)

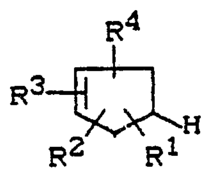

(XI)

in welcher

$R^1$, $R^2$, $R^3$ und $R^4$ die oben angegebene Bedeutung haben gefunden, das dadurch gekennzeichnet ist, daß man Dicarbonylverbindungen der allgemeinen Formel (XIIIa) oder (XIIIb)

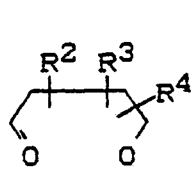

(XIIIa)

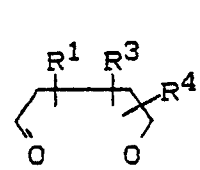

(XIIIb)

in welcher

$R^1$, $R^2$, $R^3$ und $R^4$ die oben angegebene Bedeutung haben in inerten Lösemitteln, gegegenenfalls in Anwesenheit von Titantetrachlorid mit Aminen der allgemeinen Formeln (XIVa) oder (XIVb)

$H_2N\text{-}R^1$ (XIVa) $H_2N\text{-}R_2$ (XIVb)

in welcher

$R^1$ und $R^2$ die oben angegebene Bedeutung haben,

umsetzt.

Das erfindungsgemäße Verfahren kann beispielsweise durch folgendes Formelschema verdeutlicht werden:

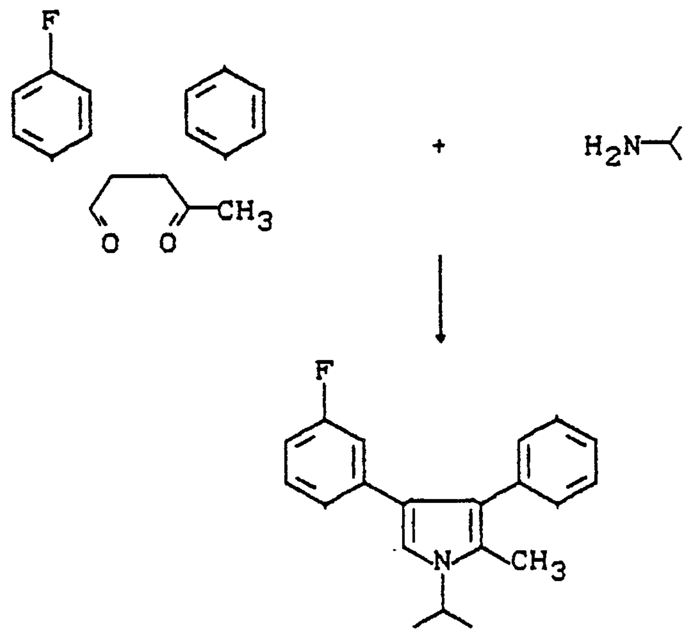

Als Lösemittel eignen sich hierbei die üblichen organischen Lösemittel, die unter den Reaktionsbedingungen stabil sind. Hierzu gehören bevorzugt Alkohole wie Methanol und Ethanol, Wasser, Aceton, Ether wie Diethylether, Dioxan oder Tetrahydrofuran oder Kohlenwasserstoffe wie Benzol, Toluol, Xylol oder Hexan. Ebenso ist es möglich, Gemische der genannten Lösemittel einzusetzen. Besonders bevorzugt wird ein Gemisch aus Ethanol und Wasser.

Die Reaktion wird in einem Temperaturbereich von -40° C bis +100° C, bevorzugt von 0° C bis +80° C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Amin im allgemeinen in einer Menge von 1 bis 10, bevorzugt von 1 bis 5 Mol, bezogen auf 1 Mol der Dicarbonylverbindung eingesetzt.

Die als Ausgangsstoffe eingesetzten Dicarbonylverbindungen der allgemeinen Formel (XIIIa) und (XIIIb) sind an sich bekannt.

Die Amine der allgemeinen Formel (XIVa) und (XIVb) sind bekannt oder können nach bekannter Methode hergestellt werden.

Es wurde außerdem ein neues Verfahren zur Herstellung der Dicarbonylverbindungen der allgemeinen Formel (XIIIa) und (XIIIb)

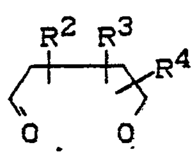

(XIIIa)

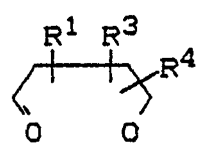

(XIIIb)

in welcher

$R^1$, $R^2$, $R^3$ und $R^4$ die oben angegebene Bedeutung haben gefunden, das dadurch gekennzeichnet ist, daß man Verbindungen der allgemeinen Formel (XVa) und (XVb)

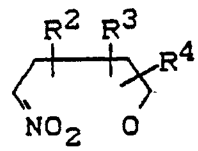

(XVa)

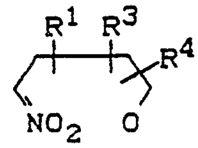

(XVb)

in welcher
$R^1$, $R^2$, $R^3$ und $R^4$ die oben angegebene Bedeutung haben
zunächst mit Basen und anschließend mit Mineralsäuren oder Kaliumpermanganat versetzt.

Das erfindungsgemäße Verfahren kann beispielsweise durch folgendes Formelschema verdeutlicht werden:

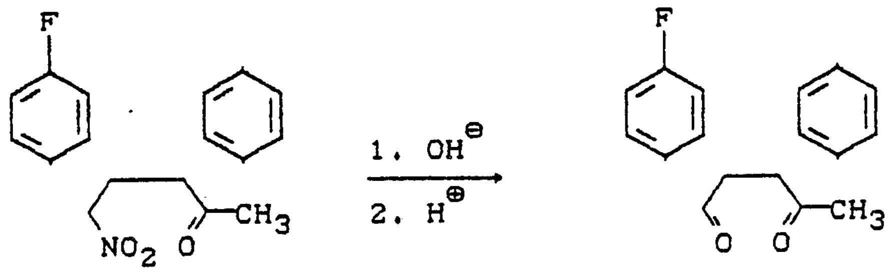


Als Lösemittel eignen sich hierbei die üblichen organischen Lösemittel, die unter den Reaktionsbedingungen stabil sind. Hierzu gehören bevorzugt Alkohole wie Methanol und Ethanol, Wasser, Aceton, Ether wie Diethylether, Dioxan oder Tetrahydrofuran. Ebenso ist es möglich, Gemische der genannten Lösemittel einzusetzen. Besonders bevorzugt wird ein Gemisch aus Ethanol und Wasser.

Als Basen werden im allgemeinen Metallhydroxide oder Metallalkoholate verwendet, wie Natrium- oder Kaliumhydroxid, Namethanolat oder Naethanolat. Bevorzugt wird Natrium- oder Kaliumhydroxid verwendet.

Als Mineralsäuren werden Chlorwasserstoffsäure, Bromwasserstoffsäure oder Schwefelsäure verwendet.

Die Reaktion wird in einem Temperaturbereich von -40°C bis +100°C, bevorzugt von -5°C bis +10°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die als Ausgangsstoffe eingesetzten Verbindungen der allgemeinen Formel (XVa) und (XVb) sind an sich bekannt.

Es wurde außerdem ein verbessertes Verfahren zur Herstellung der Verbindungen der allgemeinen Formel (XVa) und (XVb)

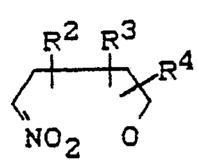

(XVa)

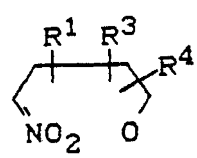

(XVb)

in welcher
$R^1$, $R^2$, $R^3$ und $R^4$ die oben angegebene Bedeutung haben gefunden,
das dadurch gekennzeichnet ist, daß man Verbindungen der allgemeinen Formel (XVI)

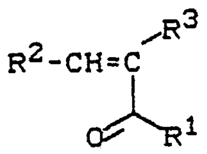

(XVIa)

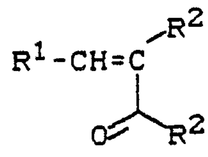

(XVIb)

in welcher

$R^1$, $R^2$ und $R^3$ die oben angegebene Bedeutung haben in Anwesenheit von Basen mit Nitromethan der Formel (XVII)

$CH_3-NO_2$ (XVII)

umsetzt.

Das erfindungsgemäße Verfahren kann beispielsweise durch folgendes Formelschema verdeutlicht werden:

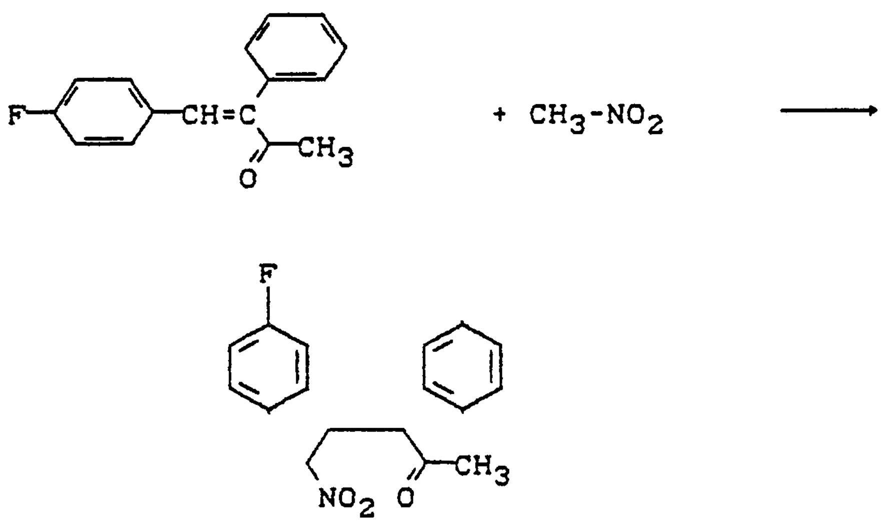

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff, oder Alkohole wie Methanol oder Ethanol oder Acetonitril. Ebenso ist es möglich, Gemische der genannten Lösemittel einzusetzen. Besonders bevorzugt wird wasserfreies Acetonitril verwendet.

Als Basen werden im allgemeinen Amine, Amidine, Guanidine oder Metallhydroxide verwendet. Bevorzugt wird 1,8-Diazobicyclo[5.4.0]-7-undecen verwendet.

Die Reaktion wird in einem Temperaturbereich von -40°C bis +80°C, bevorzugt von 0°C bis +30°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die als Ausgangsstoffe eingesetzten Verbindungen der allgemeinen Formel (XVIa) und (XVIb) sind bekannt (J. Org. Chem. Vol. 38, Nr. 9, 1747, 1973).

Die nach dem erfindungsgemäßen Verfahren hergestellten 2-substituierten Pyrrole der allgemeinen Formel (I) können als Inhibitoren der 3-Hydroxy-3-methyl-glutaryl-Coenzym A (HGM-CoA) Reduktase und auch als Inhibitoren der Cholesterolbiosynthese verwendet werden. Sie können deshalb zur Behandlung von Hyperlipoproteinämie, Lipoproteinämie oder Arteriosklerose eingesetzt werden.

<u>Herstellungsbeispiele</u>

Beispiel 1

1-(4′-Fluorphenyl)-2-phenyl-buten-3-on

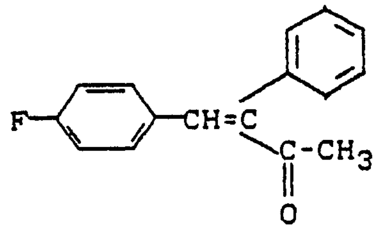

Eine Lösung von 26,8 g (0,2 mol) Phenylaceton und 24,8 g (0,2 mol) 4-Fluor-benzaldehyd in 200 ml Toluol wird mit 2 ml Piperidin und 2,4 ml Eisessig am Wasserabscheider gekocht. Nach 2,5 h haben sich ca. 3,5 ml Wasser abgeschieden. Man läßt abkühlen, engt im Vakuum ein, verrührt den Kristallbrei mit n-Hexan und saugt ab.
Ausbeute: 32,8 g (68% der Theorie)
Schmp.: 80,5 - 82 °C
$^1$H-NMR ($CDCl_3$): $\delta$ = 2,3 (s, 3H, $-CH_3$); 6,8 - 7,4 (m, 9H, Aromaten-H); 7,6 (s, 1H, Olefin-H).

Beispiel 2

2-(4′-Fluorphenyl)-1-nitro-3-phenyl-pentan-4-on

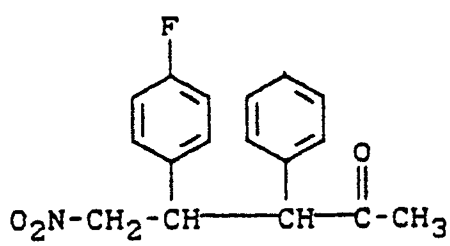

Zu einer Lösung von 15,3 g (64 mmol) 1-(4′-Fluorphenyl)-2-phenyl-buten-3-on (Beispiel 1) und 11,7 g (191 mmol) Nitromethan in 200 ml Acetonitril tropft man bei 0 °C 7,55 g (48 mmol) DBU in 10 ml Acetonitril und rührt 1 h bei Raumtemperatur. Danach wird wieder auf 0 °C gekühlt und weitere 3,52 g (22,4 mmol) DBU in 10 ml Acetonitril zugetropft. Nach Rühren über Nacht bei Raumtemperatur wird das Acetonitril im Vakuum abgezogen, mit 10 ml 0,5 M Salzsäure versetzt, dreimal mit 100 ml Chloroform extrahiert, die vereinigten organischen Phasen über Natriumsulfat getrocknet und im Vakuum eingeengt. Der Rückstand (22 g braunes Öl) wird über 200 g Kieselgel mit Petrolether / Methylenchlorid (3:1) filtriert. Ausbeute: 12 g (62% der Theorie) gelbliches Öl (Diastereomerengemisch)
$^1$H-NMR ($CDCl_3$): $\delta$ = 1,9 und 2,1 (zwei s, 3H, $CH_3$); 4,0 - 4,4 und 4,8 (m, 4H, $-CH_2$ und C-H); 6,8 - 7,5 (m, 9 H, Aromaten).

Durch zweimaliges Umkristallisieren aus Methylenchlorid / Petrolether wird ein Diastereomeres in reiner Form gewonnen.
Schmp.: 172 °C
$^1$H-NMR ($CDCl_3$): $\delta$ = 1,9 (s, 3H, $CH_3$); 4,1 - 4,4 (m, 4H, $CH_2$, CH); 7,0 (t, 2H, ortho-H zu Fluor); 7,2 - 7,5 (m, 7H, Aromaten).

Beispiel 3

2-(4-Fluorphenyl)-5-methyl-4-oxo-3-phenyl-pentanal (3a)

1-Ethoxy-2-(4′-fluorphenyl)-4-methyl-3-phenyl-1,2-dihydro-furan (3b)

1,4-Diethoxy-2-(4′-fluorphenyl)-5-methyl-3-phenyl-tetrahydrofuran (3c)

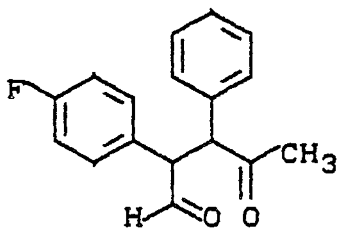

3a

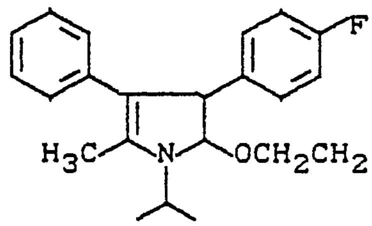

3b

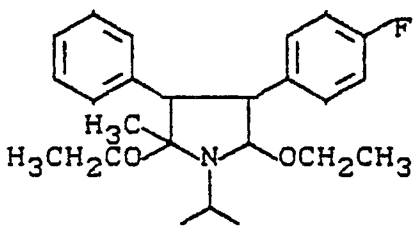

3c

Zu einer Lösung von 5,73 g (19 mmol) 2-(4′-Fluorphenyl)-1-nitro-3-phenyl-pentan-4-on (Beispiel 2) in 100 ml Ethanol gibt man eine Lösung von 1,8 g (46 mmol) Natriumhydroxid in 10 ml Wasser. Diese Lösung wird nun bei 0° C unter Rühren zu einer Lösung von 10,7 g (105 mmol) 96%iger Schwefelsäure in 50 ml Wasser und 50 ml Ethanol getropft. Das Ethanol wird im Vakuum abgezogen, der wäßrige Rückstand dreimal mit Chloroform extrahiert und die vereinigten organischen Phasen werden über Natriumsulfat getrocknet und zu einem braunen Öl (5,5 g) eingeengt. Eine Säulenchromatographie (180 g Kieselgel 230-400 mesh, Ø 5 cm, Petrolether-Methylenchlorid 1:1) ergibt drei Fraktionen:

Fraktion 3a: 1,3 g (25% der Theorie) gelbliches Öl $R_f$ (Toluol) = 0,3

$^1$H-NMR ($CDCl_3$): $\delta$ = 2,2 (s, 3H, $CH_3$); 4,3 (d, 1H); 4,5 (d, 1H); 6,8 - 7,2 (m, 9H, Aromaten-H); 9,7 (s, 1H, Aldehyd-H).
MS: m/e = 270 (9%, $M^+$), 242 (35%, M-CO)

Fraktion 3b: 0,41 g (7% der Theorie) gelbliches Öl $R_f$ (Toluol) = 0,7

$^1$H-NMR ($CDCl_3$): $\delta$ = 1,3 (t, 3H, $CH_2$-$CH_3$); 2,2 (s, 3H, $CH_3$); 3,6 und 3,9 (zwei m, 2H, $CH_2$-$CH_3$); 4,2 (m, 1H; -C-H); 5,2 (d, 1H).
MS: m/e = 298 (100%, $M^+$), 43 (70%, $CH_3CO^+$)

Fraktion 3c: 0,93 g (14% der Theorie) bräunliches Öl, $R_f$ (Toluol) = 0,6

$^1$H-NMR ($CDCl_3$): $\delta$ = 1,2 (m, 6H, $CH_2$-$CH_3$); 3,2 (d, 1H, CH); 3,4 - 3,9 (vier m, 4H, $CH_2$-$CH_3$); 4,0 (dd, 1H, CH); 5,1 (d, 1H, CH); 6,9 - 7,4 (m, 9H, Aromaten-H).
MS: m/e = 299 (15%, M-$OC_2H_5$), 256 (85%, M-$OC_2H_5$-$CH_3CO$)

Beispiel 4

4-(4-Fluorphenyl)-1-isopropyl-2-methyl-4-phenyl-pyrrol

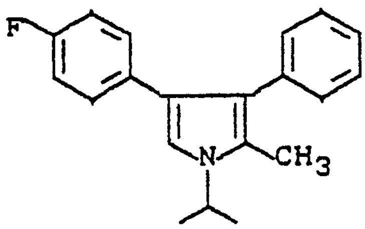

1,2 g (4,4 mmol) 3a und 0,35 g (1,18 mmol) 3b und 0,8 g (2,3 mmol) 3c, zusammen 7,88 mmol werden mit 4 g (47,4 mmol) 70%igen Isopropylamin und 1 g (16,6 mmol) Eisessig in 50 ml Toluol 2 h am Wasserabscheider gekocht. Man gibt weitere 4 g 70%iges Isopropylamin zu und erhitzt noch 2 h. Nach dem Abkühlen gibt man 50 ml Toluol zu, wäscht mit gesättigter Natriumhydrogencarbonat-Lösung und gesättigter Natriumchlorid-Lösung und trocknet über Kaliumcarbonat. Nach Abziehen des Lösemittels bleiben 2,0 g oranges Öl, die an 50 g Kieselgel 230-400 mesh mit Petrolether-Methylenchlorid 5:1 chromatographiert werden.
Ausbeute: 1,1 g (48% der Theorie) farblose Kristalle
Schmp.: 111 °C
$^1$H-NMR ($CDCl_3$): $\delta$ = 1,5 (d, 6H, isopropyl-$CH_3$); 2,2 (s, 3H, -$CH_3$); 4,35 (sept, 1H, isopropyl-H); 6,8 - 7,3 (m, 10 H, Pyrrol-H und Aromaten-H).

Beispiel 5

(E)-3-[3-(4-Fluorphenyl)-1-isopropyl-5-methyl-4-phenylpyrrol-2-yl]-prop-2-enal

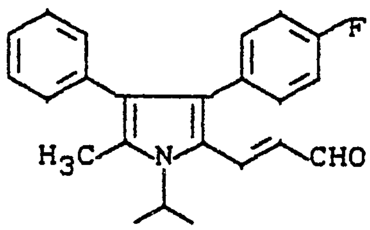

Zu einer Lösung von 0,3 ml (3,3 mmol) Phosphoroxychlorid in 10 ml trockenem Acetonitril tropft man bei -5° C eine Lösung von 0,35 g (3,2 mmol) 90% Dimethylaminoacrolein in 8 ml Acetonitril. Bei der selbem Temperatur werden portionsweise 0,75 g (2,56 mmol) 4 zugegeben. Dann läßt man auf Raumtempertaur erwärmen und rührt schließlich noch 1 h bei 50° C. Den Ansatz gibt man nun bei 10° C zu einer Emulsion aus 30 ml Wasser, in dem 1,8 g Natriumhydroxid gelöst sind, und 30 ml Toluol und rührt 30 min. kräftig durch. Die organische Phase wird abgetrennt, mit gesättigter Natriumchlorid-Lösung gewaschen und über Natriumsulfat und Kaliumcarbonat getrocknet. Nach dem Einengen erhält man ein Öl, das bald kristallisiert.
Ausbeute: 0,87 g (98% der Theorie) gelber Feststoff
Schmp.: 168° C

Beispiel 6

Methyl-(E)-7-[3-(4-fluorphenyl)-1-isopropyl-5-methyl-4-phenyl-pyrrol-2-yl]-5-hydroxy-3-oxo-hept-6-enoat

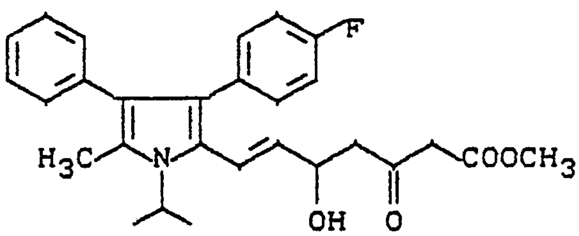

Unter Argon gibt man zu einer Suspension von 118 mg (3,92 mmol) 80%igem Natriumhydrid in 5 ml wasserfreiem THF bei 0° C 427 mg (3,67 mmol) Acetesssigsäuremethylester. Nach 15 min. werden bei derselben Temperatur in nerhalb von 10 min. 2.7 ml (4,3 mmol) 15%iges Butyllithium in Hexan zugetropft und 15 min. nachgerührt. Jetzt tropft man eine Lösung von 0,85 g (2,45 mmol) 5 in 10 ml THF zu und rührt weitere 15 min. bei dieser Temperatur. Anschließend werden vorsichtig 10 ml 1 M Essigsäure zugetropft, die Mischung dreimal mit 30 ml Ether extrahiert, die vereinigten organischen Phasen mit gesättigter Natriumchlorid-Lösung gewaschen und über Natriumsulfat und Kaliumcarbonat getrocknet. Nach Abziehen des Lösemittels erhält man 1,3 g eines rötlichen Öls, das in einer Säule (30 g Kieselgel 290-400 mesh, Ø 2,5 cm, Petrolether-Essigester 4:1) chromatographiert wird.
Ausbeute: 0,55 g (48% der Theorie) gelbliches Öl.
$R_f$ = 0,4 (Petrolether-Essigester 3:1)

Beispiel 7

Methyl-erythro-(E)-3,5-dihydroxy-7-[3-(4-fluorphenyl-1-isopropyl-5-methyl-4-phenyl-pyrrol-2-yl]hept-6-enoat

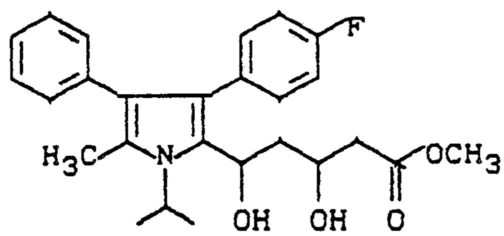

Zu einer Lösung von 0,3 g (0,65 mmol) 6 in 10 ml wasserfreiem THF tropft man bei Raumtemperatur unter Argon 0,78 ml (0,78 mmol) 1 M Triethylboran-Lösung in Hexan, leitet 5 min lang Luft durch die Lösung und kühlt auf -78° C ab. Dann werden 30 mg (0,8 mmol) Natriumborhydrid und vorsichtig 0,43 ml MeOH zugegeben, daß die Temperatur -65° C nicht übersteigt. Es werden weitere 15 min. bei -75° C und 15 min. bei -30° C gerührt. Bei 0° C tropft man dann eine Lösung von 2,2 ml 30%igem Wasserstoffperoxid in 6 ml Wasser zu, läßt auf Raumtemperatur erwärmen, extrahiert dreimal mit Essigester, wäscht die organische Phase mit gesättigter Natriumhydrogencarbonat-Lösung, trocknet über Natriumsulfat und engt im Vakuum ein. Das zurückbleibende gelbe Öl (315 mg) wird in einer Säule (15 g Kieselgel 230-400 mesh, ⌀ 1,5 cm) mit Petrolether-Essigester (2:1) chromatographiert.

Ausbeute: 188 mg (62% der Theorie) farblosen Schaum.

$^1$H-NMR ($CDCl_3$): $\delta$ = 1,4 - 1,6 (m, 8H, Isopropyl-$CH_3$ und CH(OH)-$CH_2$-CHOH); 2,3 (s, 3H, 5′-$CH_3$); 2,45 (m, 2H, $CH_2$-$CO_2$-$CH_3$); 3,0 (b, 1H, OH); 3,6 (b, 1H, OH); 3,7 (s, 3H, O-$CH_3$); 4,15 (m, 1H, HO-CH); 4,4 (m, 1H, HO-CH) 4,65 (sept, 1H, Isopropyl-H); 5,3 (dd, 1H, Olefin-H); 6,7 (d, 1H, Olefin-H); 6,8 - 7,2 (m, 9H, Aromaten-H).

Beispiel 8

1-(4-Fluorphenyl)-4-methyl-pent-1-en-3-on

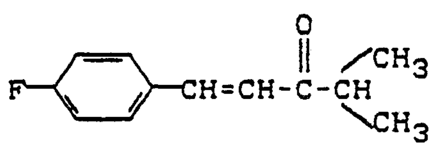

Zu 198,4 g (1,6 mol) frisch destilliertem 4-Fluorbenzaldehyd und 137,6 g (1,6 mol) Methylisopropylketon in 300 ml Methanol tropft man 75 ml 15%ige Kalilauge und rührt über Nacht bei Raumtemperatur. Dann wird mit 10 ml Essigsäure neutralisiert, 1 l Wasser zugesetzt und mit zwei 500 ml Portionen Ether extrahiert. Die vereinigten organischen Phasen werden mit 500 ml gesättigter Natriumchlorid-Lösung gewaschen und über Natriumsulfat getrocknet. Nach Abziehen des Lösemittels destilliert man im Hochvakuum.

Ausbeute: 198,6 g (65% der Theorie) gelbliches Öl

Kp.: 103° C (0.3 mbar)

$^1$H-NMR ($CDCl_3$): $\delta$ = 1,2 (d, 6H, $CH_3$); 2,9 (sept, 1H, CH-$(CH_3)_2$); 6,8 (d, 1H, Olefin-H); 7,1 (m, 2H, Aromaten-H); 7,6 (m, 3H, Aromaten-H + Olefin-H).

Beispiel 9

2-(4'-Fluorphenyl)-5-methyl-1-nitro-hexan-4-on

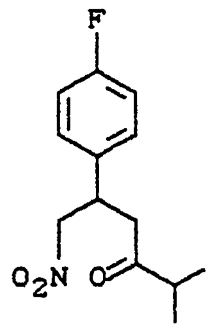

Zu einer Lösung von 50 g (0,26 mol) 1-(4'-Fluorphenyl)-4-methyl-pent-1-en-3-on (Beispiel 8) und 19,0 g (0,31 mol) Nitromethan in 150 ml Acetonitril tropft man bei 0 °C 18,9 g (0,13 mol) Diazabicyclo-undecen in 100 ml Acetonitril zu und rührt anschließend 1 h bei Raumtemperatur. Es wird mit 250 ml 0,5 M Salzsäure versetzt, dreimal mit je 200 ml Chloroform extrahiert, die vereinigten organischen Phasen über Natriumsulfat getrocknet und eingeengt. Der Rückstand (66 g braunes Öl) wird in einer Säule (450 g Kieselgel, ∅ 5 cm, Petrolether-Essigester 10:1) chromatographiert. Man erhält zwei Zonen:

1. 8,3 g (20% der Theorie) bräunliches Öl

$R_f$ = 0,5 (Petrolether-Essigester 5:1)

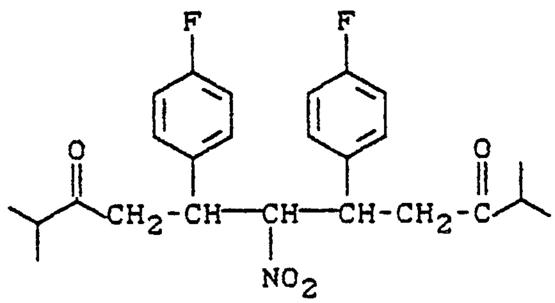

$^1$H-NMR ($CDCl_3$): $\delta$ = 0,8 - 1,1 (vier d, 12H, isopropyl-$CH_3$); 2,3 - 3,1 (m, 6H, $CH_2$ und isopropyl-H); 3,5 - 3,7 (m, 2H, C-H); 5,3 (m, 1H, $O_2N$-C-H); 6,8 - 7,3 (m, 8H, Aromaten).

2. 26,7 g (41% der Theorie) bräunliches Öl $R_f$ = 0,36 (Petrolether-Essigester 5:1)

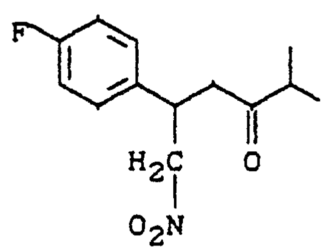

$^1$H-NMR ($CDCl_3$): $\delta$ = 1,0 - 1,1 (zwei d, 6H, isopropyl-$CH_3$); 2,5 (sept, 1H, isopropyl-H); 2,9 (d, 2H, $CH_2$); 4,0 (m, 1H); 4,65 (m, 2H, $CH_2$).

Beispiel 10

2-(4′-Fluorphenyl)-5-methyl-4-oxo-hexanal

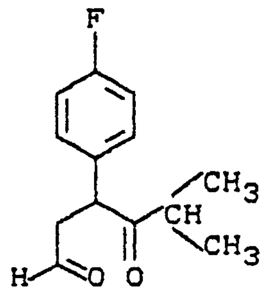

Eine Lösung von 20 g (79 mmol) 1 in 50 ml Ethanol wird mit einer Lösung von 3,8 g (95 mmol) Natriumhydroxid in 50 ml Wasser vereinigt. Man trennt von etwas Natriumchlorid ab und tropft das Filtrat bei 0° C unter Rühren in 73 ml (219 mmol) 3 M Schwefelsäure ein. Anschließend wird das Ethanol abrotiert und der wäßrige Rückstand mit ca. 100 ml Chloroform durchgeschüttelt. Die organische Phase extrahiert man mit gesättigter Nahydrogencarbonat-Lösung, trocknet über Natriumsulfat und engt zu einem braunen Öl (17 g ) ein. Säulenchromatographie (400 g Kieselgel 230-400 mesh, ∅ 5 cm, Petrolether-Essigester 20:1) ergibt 8,1 g (46% der Theorie) rötliches Öl.
$^1$H-NMR ($CDCl_3$): $\delta$ = 1,1 (dd, 6H, Isopropyl-$CH_3$); 2,6 (m, 2H, Isopropyl-H und $CH_2$); 2,9 (dd, 1H, $CH_2$); 4,3 (dd, 1H, CH); 7,0 -7,3 (m, 4H, Aromaten-H).

Beispiel 11

1,2-Diisopropyl-3-(4′-fluorphenyl)-pyrrol

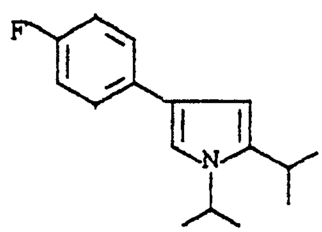

8,1 g (36,4 mmol) 2, 23 ml (218 mmol) 70%iges Isopropylamin und 4,2 (73 mmol) Essigsäure werden in 300 ml Toluol 3 h am Wasserabscheider gekocht. Dann wird einrotiert, der Rückstand in 300 ml Chloroform aufgenommen, mit 300 ml 1 N Salzsäure und 300 ml gesättigter Natriumhydrogencarbonat-Lösung gewaschen, über Natriumsulfat getrocknet und eingeengt. Das zurückbleibende Öl (9, 4 g) wird mit Petrolether-Essigester (4:1) über 150 g Kieselgel (230-400 mesh) filtriert. Das Produkt kristallisiert nach Abziehen des Lösemittels.
Ausbeute: 7,1 g (80% der Theorie) farblose Kristalle
Schmp.: 51° C
$^1$H-NMR ($CDCl_3$: $\delta$ = 1,3 (d, 6H, C-isopropyl-$CH_3$); 1,5 (d, 6H, N-isopropyl-$CH_3$); 2,9 (sept, 1H, C-isopropyl-H); 4,3 (sept, 1H, N-isopropyl-H); 6,1 (d, 1H, 3-H); 6,9 (d, 1H, 5-H); 7,0 (t, 2H, 3′-H); 7,4 (m, 2H, 2′ -H).

Beispiel 12

(E)-3-[3′-(4″-Fluorphenyl)-1′,2′-diisopropyl-pyrrol-2-yl]prop-2-enal

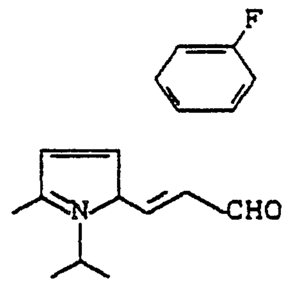

Analog Beispiel 5 wird aus 6,6 ml (72 mmol) Phosphoroxychlorid in 50 ml trockenem Acetonitril, 6,8 g (69 mmol) Dimethylaminoacrolein in 40 ml Acetonitril und 6 g (24,5 mmol) der Verbindung aus Beispiel 11 die Titelverbindung hergestellt. Die Aufarbeitung erfolgt nach üblichen Methoden in einer Emulsion von 17 g Natriumhydroxid, 250 ml Wasser und 250 ml Toluol.
Säulenchromatographie: 250 g Kieselgel (230-400 mesh), ⌀ 4 cm, Methylenchlorid
Ausbeute: 5,1 g (70% der Theorie) rötliches Öl.
$^1$H-NMR ($CDCl_3$): $\delta$ = 1,3 (d, 6H, C-isopropyl-$CH_3$); 1,6 (d, 6H, N-isopropyl-$CH_3$); 3,1 (m, 1H, C-isopropyl, H); 4,7 (sept, 1H, N-isopropyl, H); 6,1 (m, 2H, 4′-H und Olefin-H); 7,1 (t, 2H, 3″-H); 7,3 (m, 2H, 2″-H); 7,5 (d, 1H, Olefin-H).

Beispiel 13

Methyl-(E)-7-[3′-(4″-fluorphenyl-1′,5′-diisopropylpyrrol-2′-yl]-5-hydroxy-3-oxo-hept-6-enoat

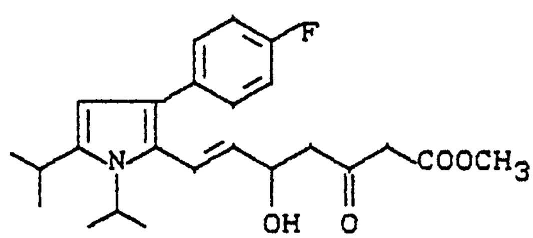

Analog Beispiel 6 wird aus 0,12 g (4 mmol) 80%igen Natriumhydrid, 0,426 g (3,7 mmol) Acetessigsäuremethylester, 2,7 ml (4,4 mmol) 15%igem Butyllithium und 1,0 g (3,34 mmol) der Verbindung aus Beispiel 12 die Titelverbindung hergestellt. Man erhält 1,1 g Rohrprodukt. Säulenchromatographie: 50 g Kieselgel (230-400 mesh, ⌀ 2,5 cm, Methylenchlorid)
Ausbeute: 0,61 g (44% der Theorie) rötliches Öl
$^1$H-NMR ($CDCl_3$); charakteristische Signale:
$\delta$ = 1,3 (d, 6H); 1,5 (d, 6H); 2,6 (m, 2H); 3,0 (sept, 1H); 3,7 (s, 3H); 4,5 (sept, 1H); 4,6 (m, 1H); 5,4 (dd, 1H); 6,7 (d, 1H); 6,7 (d, 1H); 7,0 (t, 2H); 7,3 (m, 2H).

Beispiel 14

Methyl-erythro-(E)-3,5-dihydroxy-7-[1′,5′-diisopropyl-3′-(4″-fluorphenyl)-pyrrol-2′-yl]-hept-6-enoat

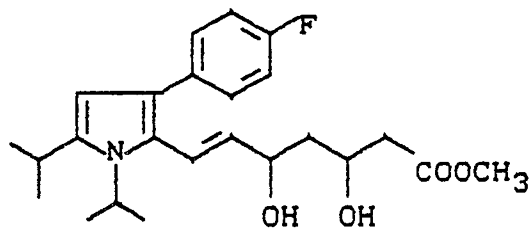

Analog Beispiel 7 erhält man aus 0,5 g (1,2 mmol) der Verbindung aus Beispiel 13, 1,45 ml (1,45 mmol) 1 M Triethylboran in Hexan, 56 mg (1,48 mmol) Natriumborhydrid und 4 ml 30%igem Wasserstoffperoxid in 15 ml Wasser die Titelverbindung.
Ausbeute: 285 mg (57% der Theorie) gelbliches Öl.
$^1$H-NMR ($CDCl_3$): δ = 1,3 (d, 6H, C-isopropyl-$CH_3$); 1,5 (m, 8H, N-isopropyl-$CH_3$ und CH(OH)-$\underline{CH_2}$-CHOH); 2,5 (m, 2H, $\underline{CH_2}$-$CO_2$-$CH_3$); 3,0 (m, 2H, C-isopropyl-H und OH); 3,6 (b, 1H, OH), 3,7 (s, 3H, O-$CH_3$); 4,2 (m, 1H, HO-CH); 4,4 (m, 1H, HO-CH); 4,55 (sept, 1H, N-isopropyl-H); 5,5 (dd, 1H, Olefin-H); 5,9 (s, 1H, 4′-H); 7,0 (t, 2H, 3″-H); 7,3 (m, 2H, 2″-H).

Beispiel 15

(E/Z)-1-(4-Fluorphenyl)-4-methyl-2-phenyl-penten-3-on

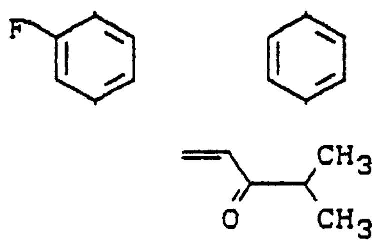

Analog Beispiel 1 erhält man aus 31 g (0,25 mol) 4-Fluorbenzaldehyd und 40,6 g (0,25 mol) 2-Methyl-4-phenyl-butan-3-on, nach Reinigung durch Destillation 56,8 g.
Ausbeute: 85 % der Theorie
Kp.: 140 °C (0,2 mbar)
Fp.: 46 °C
$^1$H-NMR ($CDCl_3$): δ = 1,0; 1,1 (2d, 6H, $CH_3$); 2,6; 3,1 (2 sept, 1H, CH); 6,8-7,6 (m, 10 H, Aromaten-H, CH) ppm.

Beispiel 16

5-(4-Fluorphenyl)-2-methyl-6-nitro-4-phenyl-hexan-3-on

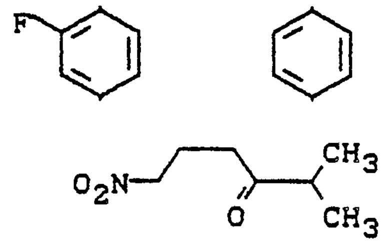

Analog Beispiel 2 erhält man aus 5,0 g (18,6 mmol) 15, 1,36 g (22,4 mmol) Nitromethan und 1,46 g (9,3 mmol) DBU 1,9 g.
Ausbeute: 31 % der Theorie
Schmp.: 175° C
$^1$H-NMR ($CDCl_3$): $\delta$ = 0.65; 0,75 (2d, 6H, $CH_3$); 2,4 (sept, 1H, $CH(CH_3)_2$)); 4,1-4,5 (m, 4H, CH, $CH_2$); 6,9-7,5 (m, 9H, Aromaten-H) ppm.

Beispiel 17

2-(4-Fluorphenyl)-5-methyl-4-oxo-3-phenyl-hexanol

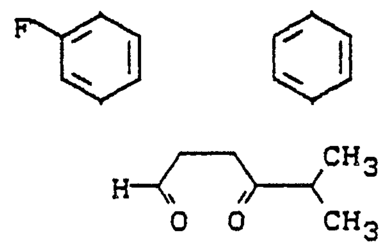

Analog Beispiel 3 erhält man aus 1,8 g (5,46 mmol) 16, 1,1 g Öl.
Ausbeute: 68 % der Theorie

Beispiel 18

4-(4-fluorphenyl)-1,2-Diisopropyl-3-phenyl-pyrrol

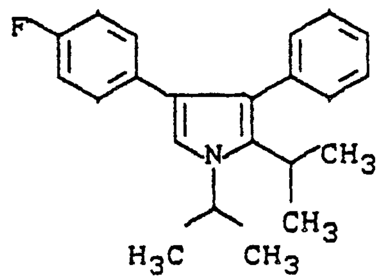

Analog Beispiel 4 erhält man aus 1,1 g (3,68 mmol) 17, 3,1 g (36,8 mmol) Isopropylamin und 2,0 g (33,1 mmol) Essigsäure, 1,0 g braunes Öl, das an 20 g Kieselgel 230-400 mesh mit Petrolether/Dichlormethan 3:1 während der Chromatographie propyliert wird.
Ausbeute: 0,26 g (22 % der Theorie)
Schmp.: 150 °C
$^1$H-NMR ($CDCl_3$): $\delta$ = 1,25 (d, 3H, $CH_3$); 1,55 (d, 3H, $CH_3$); 3,2 (sept, 1H, $CH(CH_3)_2$); 4,5 (sept, 1H, $CH(CH_3)_2$); 6,75-7,3 (m, 10H, Aromaten-H) + 5-H).

Beispiel 19

(E)-3-[4-(4-fluorphenyl)-1,2-Diisopropyl-3-phenyl-pyrrol-2-yl]-prop-2-enal

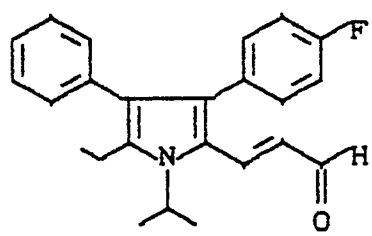

Analog Beispiel 5 erhält man aus 3,7 g (11,5 mmol) 18, 3,6 g (32,2 mmol) Dimethylaminoacrolein und 3,1 ml (34,3 mmol) Phosphoroxychlorid 2,25 g gelbe Kristalle.
Ausbeute: 52 % der Theorie
Schmp. 187 °C

Beispiel 20

Methyl-(E)-7-[4-(4-fluorphenyl)-1,2-diisopropyl-3-phenyl-pyrrol-2-yl]-5-hydroxy-3-oxo-hept-6-enoat

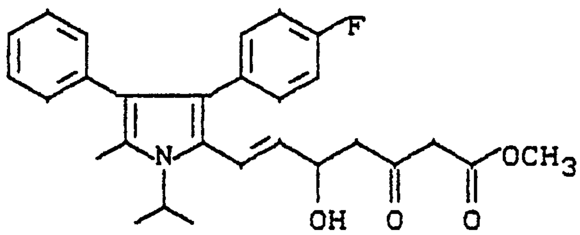

Analog Beispiel 6 erhält man aus 2,1 g (5,7 mmol) 19, 2,0 g rotes Öl. Chromatographie an 60 g Kieselgel 230-400 mesh (Petrolether-Essigester 5:1) liefert 0,67 g rotes Öl.
Ausbeute: 24 % der Theorie

Beispiel 21

Methyl-erythro-(E)-3,5-dihydroxy-7-[4-(4-fluorphenyl)-1,2-diisopropyl-3-phenyl-pyrrol-2-yl]-hept-6-enoat

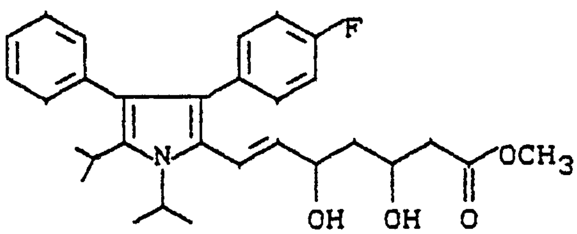

Analog Beispiel 7 erhält man aus 0,65 g (1,28 mmol) 20, 0,7 g gelbes Öl, das an 50 g Kieselgel 230-400 mesh (Petrolether-Essigester 2:1) chromatographiert wird.
Ausbeute: 460 mg (73 % der Theorie)
$^1$H-NMR ($CDCl_3$): $\delta$ = 1,25 (d, 6H, $CH_3$); 1,4 (m, 2H, $CH_2$); 1,6 (d, 6H, $CH_3$); 2,4 (m, 2H, $CH_2$); 2,85 (b, 1H, OH); 3,25 (sept, 1H, $CH(CH_3)_2$); 3,6 (d, 1H, OH); 3,7 (s, 3H, $CH_3$); 4,1 (m, 1H, CHOH); 4,35 (m, 1H, CHOH); 4,7 (sept, 1H, $CH(CH_3)_2$); 5,2 (dd, 1H, CH); 6,65 (d, 1H, CH); 6,7-7,2 (m, 3H, Aromaten-H) ppm.

Beispiel 22

Erythro-(E)-3,5-dihydroxy-7-[4-(4-fluorphenyl)-1,2-diisopropyl-3-phenyl-pyrrol-2-yl]-hept-6-en-säure-natriumsalz

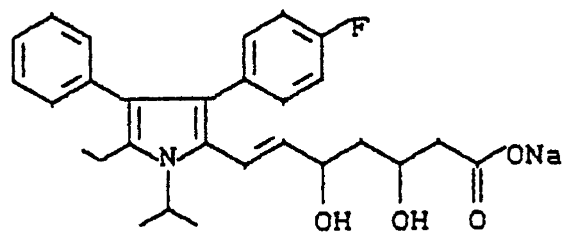

Eine Lösung von 100 mg (0,203 mmol) 21 und 182 µl (0,182 mmol) 1 N Natronlauge in 2 ml Tetrahydrofuran wird 2 h bei 25°C gerührt, das Lösungsmittel wird im Vakuum entfernt und der Rückstand über Phosphorpentoxid im Vakuum getrocknet.
Ausbeute: 100 mg (98 % der Theorie)
Schmp.: 205°C-210°C (Zers.)

Beispiel 23

4-Methyl-1-phenyl-pent-2-en-1-on

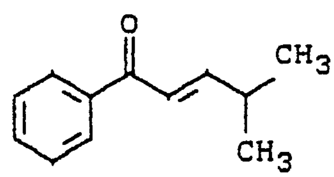

Analog Beispiel 8 erhält man 23 und 4-Methyl-1-phenyl-pent-3-en-1-on als Isomerengemisch (1:1).
Ausbeute: 40 % der Theorie
Kp.: 110°C (1,5 mbar)
$^1$H-NMR ($CDCl_3$): δ = 1,1 (d, 6H, $CH_3$); 1,7 (s, 3H, $CH_3$; 1,8 (s, 3H, $CH_3$); 2,6 (sept, 1H, CH); 3,7 (d, 2H, $CH_2$); 5,45 (m, 1H, CH); 6,8 (d, 1H, CH); 7,05 (dd, 1H, CH); 7,4-8,0 (m, 10H, Aromaten-H) ppm.

Beispiel 24

4-Methyl-3-nitromethyl-1-phenyl-pentan-on

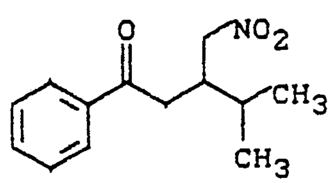

Analog Beispiel 9 erhält man aus 57 g (0,33 mol) 23, nach Chromatographie über Kieselgel (Essigester-Petrolether 1:20) 44,4 g.

Ausbeute: 57,8 % der Theorie
$^1$H-NMR ($CDCl_3$): $\delta$ = 1,0 (dd, 6H, $CH_3$); 1,9 (m, 1H, CH); 2,85 (m, 1H, CH); 3,1 (m, 2H, $CH_2C=O$); 4,5 (m, 2H, $CH_2NO_2$); 7,4-8,0 (m, 5H, Aromaten-H) ppm.

Beispiel 25

3-Isopropyl-1-phenyl-pentan-1,4-dion

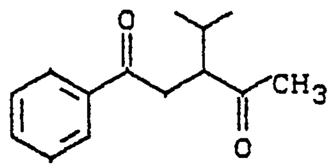

Analog Beispiel 10 erhält man aus 24,19 g (103 mmol) 24, 9,7 g Öl.

Beispiel 26

1-(4-Fluorphenyl)-4-isopropyl-2-phenyl-pyrrol

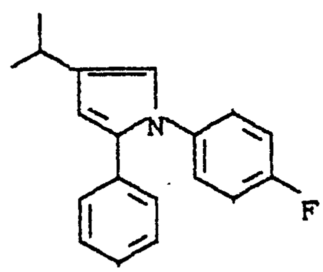

Analog Beispiel 11 erhält man aus 4,6 g 25 und 2,13 ml (22,5 mmol) 4-Fluoranilin 6,04 g.
Ausbeute: 96,2% der Theorie
$^1$H-NMR ($CDCl_3$): $\delta$ = 1,3 (d, 6H, $CH_3$); 2,9 (sept, 1H, CH); 6,3 (d, 1H, Pyrrol-3H); 6,7 (d, 1H, Pyrrol-1H); 6,9-7,3 (m, 9H, Aromaten-H) ppm.

Beispiel 27

(E)-3-[1-(4-Fluorphenyl)-4-isopropyl-2-phenyl-pyrrol-3-yl]-prop-2-enal

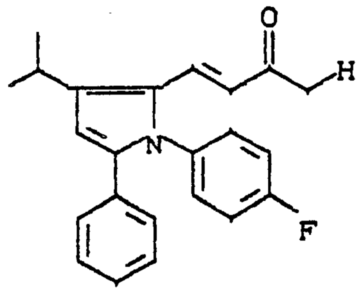

Analog Beispiel 12 erhält man aus 5,93 g (21,3 mmol) 26, 5,12 g.
Ausbeute: 72,3 % der Theorie
$^1$H-NMR ($CDCl_3$): δ = 1,35 (d, 6H, $CH_3$); 3,2 (sept, 1H, CH); 5,95 (dd, 2H, CHCHO); 6,5 (s, 1H, Pyrrol-H) 7,0-7,3 (m, 10H, CH und Aromaten-H); 9,3 (d, 1H, CHO) ppm.

Beispiel 28

Methyl-(E)-7-[1-(4-fluorphenyl)-3-isopropyl-5-phenyl-pyrrol-2-yl]-5-hydroxy-3-oxo-hept-6-enoat

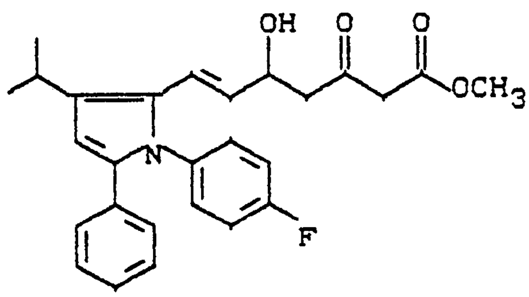

Analog Beispiel 13 erhält man aus 5,07 g (15,25 mmol) 27, 7,15 g Rohprodukt.
Rohausbeute: 100 % der Theorie
$^1$H-NMR ($CDCl_3$): δ = 1,3 (d, 6H, $CH_3$); 2,7 (m, 2H, $CH_2C=O$); 3,1 (sept, 1H, CH); 3,45 (m, 2H, $CH_2COO$); 3,7 (s, 3H, $OCH_3$); 4,55 (m, 1H, CHOH); 5,45 (dd, 14, CH-CHOH); 6,3 (d, CH, 1H); 6,35 (S, 1H, Pyrrol-H); 6,9-7,2 (m, 5H, Aromaten-H)-ppm.

Beispiel 29

Methyl-erythro-(E)-7-[1-(4-fluorphenyl)-3-isopropyl-5-phenyl-pyrrol-2-yl]-3,5-dihydroxy-hept-6-enoat

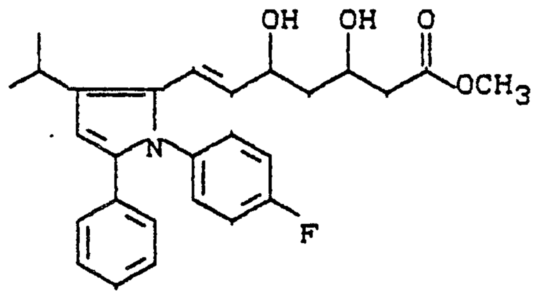

Analog Beispiel 14 erhält man aus 1,2 g (2,67 mmol) 28, nach Chromatographie an 200 g Kieselgel (Essigester-Petrolether 1:1) 430 mg.
Ausbeute: 35,8 % der Theorie
$^1$H-NMR ($CDCl_3$): $\delta$ = 1,3 (d, 6H, $CH_3$); 1,65 (m, 2H, $CH_2$); 2,5 (m, 2H, $CH_2COO$); 3,1 (sept, 1H, CH); 3,7 (s, 3H, $OCH_3$); 4,2 (m, 1H, CHOH); 4,35 (m, 1H, CHOH); 5,45 (dd, 14, CH CHOH); 6,25 (d, 1H, CH); 6,35 (s, 1H, Pyrrol-H); 6,95-7,2 (m, 9H, Aromaten-H) ppm.

**Ansprüche**

1. Verfahren zur Herstellung von substituierten Pyrrolen der Formel

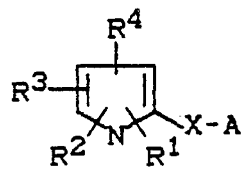

(I)

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl, oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl stehen, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben, oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
$R^3$ und $R^4$ gleich oder verschieden sind und
- für Wasserstoff oder

- für Acyl, Alkylsulfonyl oder Arylsulfonyl
stehen, wobei der Arylrest durch Alkyl oder Halogen substituiert sein kann, oder - für Aminocarbonyl, Alkylaminocarbonyl,
Dialkylaminocarbonyl, Arylaminocarbonyl oder Alkoxycarbonyl stehen, oder - für Cycloalkyl stehen, oder
- für Alkyl stehen, die substituiert sein könen durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein können,
worin
$R^5$ und $R^6$ die oben angegebene Bedeutung haben,
oder
- für Aryl stehen, die bis zu 5-fach gleich oder verschieden substituiert sein können durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben
X - für eine Gruppe der Formel $-CH_2-CH_2$ oder $-CH=CH-$ steht,
und
A - für eine Gruppe der Formel

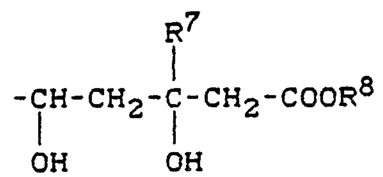 oder 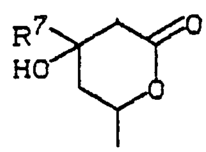 steht,

worin
$R^7$ - Wasserstoff oder Alkyl bedeutet
und
$R^8$ - Wasserstoff,
- einen physiologisch verträglichen Esterrest oder
- ein physiologisch verträgliches Kation bedeutet,
dadurch gekennzeichnet, daß man
Ketone der allgemeinen Formel

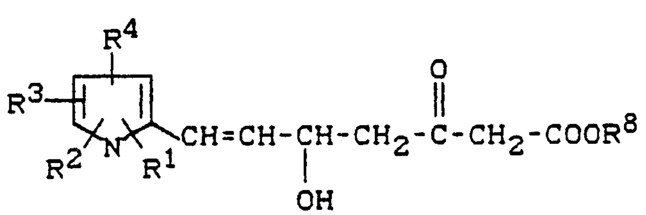

in welcher
$R^1$, $R^2$, $R^3$ und $R^4$ die oben angegebene Bedeutung haben,

und
$R^8$ - für Alkyl steht,
reduziert,
im Fall der Herstellung der Säuren die Ester verseift,
im Fall der Herstellung der Lactone die Carbonsäuren cyclisiert,
im Fall der·Herstellungder Salze entweder die Ester oder die Lactone verseift,
im Fall der Herstellung der Ethylenverbindungen (X = $-CH_2-CH_2-$) die Ethenverbindungen (X = -CH=CH-) nach üblichen Methoden hydriert,
und gegebenenfalls Isomeren trennt.

2. Verfahren nach Anspruch 1, dadurch gekennzeichnet, daß man die Reduktion im Temperaturbereich von -80 °C bis Raumtemperatur durchführt.

3. Verfahren nach den Ansprüchen 1 und 2, dadurch gekennzeichnet, daß man die Reduktion mit Metallhydriden durchführt.

4. Ketone der Formel

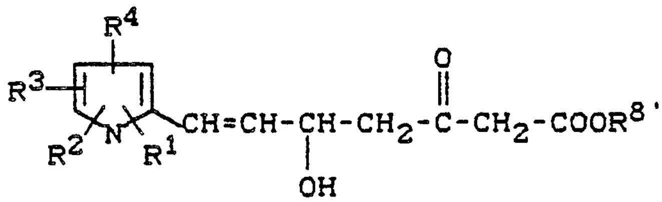

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifuormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder
durch eine Gruppe der Formel $-NR^5R^6$ substituiert sein kann,
worin
$R^5$ und $R^6$ die oben angegebene Bedeutung haben,
oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
$R^3$ und $R^4$ gleich oder verschieden sind und
- für Wasserstoff oder
- für Acyl, Alkylsulfonyl oder Arylsulfonyl stehen, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl stehen, oder
- für Cycloalkyl stehen, oder
- für Alkyl stehen, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder
- für Heteroaryl stehen, die bis zu 3-fach gleich oder verschieden durch Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein können,
worin
$R^5$ und $R^6$ die oben angegebene Bedeutung haben,
oder
- für Aryl stehen, die bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$ substituiert sein können,
worin
$R^5$ und $R^6$ die oben angegebene Bedeutung haben,
X - für eine Gruppe der Formel $-CH_2CH_2$ oder $-CH=CH$-steht,
und
$R^8$ - für Alkyl steht.

5. Verfahren zur Herstellung von Ketonen der Formel

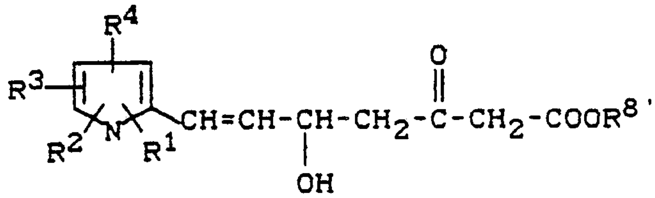

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Aryl thio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
$R^3$ und $R^4$ gleich oder verschieden sind und

- für Wasserstoff oder
- für Acyl, Alkylsulfonyl oder Arylsulfonyl stehen, wobei der Arylrest durch Alkyl oder Halogen substituiert sein können, oder
- für Aminocarbonyl, Alkylaminocarbonyl, Dialkylaminocarbonyl, Arylaminocarbonyl oder Alkoxycarbonyl stehen, oder
- für Cycloalkyl stehen, oder
- für Alkyl stehen, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder

- für Heteroaryl stehen, die bis zu 3-fach gleich oder verschieden durch Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein können,

worin

$R^5$ und $R^6$ die oben angegebene Bedeutung haben,

oder

- für Aryl stehen, die bis zu 5-fach gleich oder verschieden durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$ substituiert sein können,

worin

$R^5$ und $R^6$ die oben angegebene Bedeutung haben,

X - für eine Gruppe der Formel $-CH_2-CH_2$ oder $-CH=CH-$steht,

und

$R^8$ - für Alkyl steht,

dadurch gekennzeichnet, daß man

Aldehyde der allgemeinen Formel

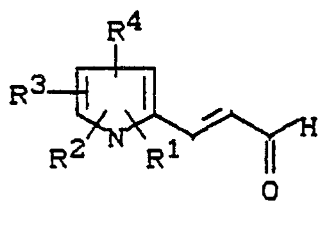

in welcher

$R^1$, $R^2$, $R^3$ und $R^4$ die oben angegebene Bedeutung haben,

in inerten Lösemitteln mit Acetessigester der allgemeinen Formel

$$H_3C-\overset{O}{\overset{\|}{C}}-CH_2-COOR^8 \quad (X)$$

in welcher

$R^8$ die oben angegebene Bedeutung hat,

in Anwesenheit von Basen umsetzt.

6. Verfahren nach Anspruch 5, dadurch gekennzeichnet, daß es im Temperaturbereich von -80 °C bis +50 °C durchgeführt wird.

7. Verfahren nach den Ansprüchen 5 und 6, dadurch gekennzeichnet, daß man den Acetessigester in einer Menge von 1 bis 2 Mol, bezogen auf 1 Mol des Aldehyds, einsetzt.

8. Aldehyde der Formel

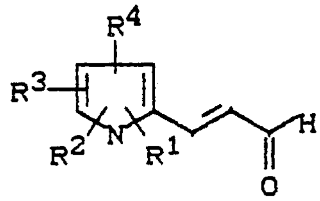

in welcher
$R^1$ - für Cycloalkyl steht, oder
- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder
- für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
$R^3$ und $R^4$ gleich oder verschieden sind und
- für Wasserstoff oder
- für Acyl, Alkylsulfonyl oder Arylsulfonyl stehen, wobei der Arylrest durch Alkyl oder Halogen substituiert sein kann, oder
- für Aminocarbonyl, Alkylaminocarbonyl, Dialkylaminocarbonyl, Arylaminocarbonyl oder Alkoxycarbonyl stehen, oder
- für Cycloalkyl stehen, oder
- für Alkyl stehen, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^5R^6$ substituiert sein können,
worin
$R^5$ und $R^6$ die oben angegebene Bedeutung haben,
oder
- für Aryl stehen, die bis zu 5-fach gleich oder verschieden substituiert sein können durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch durch eine Gruppe der Formel $-NR^5R^6$, worin $R^5$ und $R^6$ die oben angegebene Bedeutung haben.

9. Verfahren zur Herstellung von Aldehyden der Formel

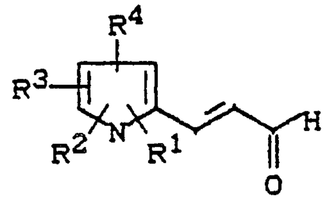

in welcher

$R^1$ - für Cycloalkyl steht, oder

- für Alkyl steht, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$, worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-oder Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^2$ - für Heteroaryl steht, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann, worin

$R^5$ und $R^6$ die oben angegebene Bedeutung haben,

oder

-für Aryl steht, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$, worin

$R^5$ und $R^6$ die oben angegebene Bedeutung haben,

$R^3$ und $R^4$ gleich oder verschieden sind und

- für Wasserstoff oder

- für Acyl, Alkylsulfonyl oder Arylsulfonyl stehen, wobei der Arylrest durch Alkyl oder Halogen substituiert sein kann, oder

- für Aminocarbonyl, Alkylaminocarbonyl, Dialkylaminocarbonyl, Arylaminocarbonyl oder Alkoxycarbonyl stehen, oder

- für Cycloalkyl stehen, oder

- für Alkyl stehen, die substituiert sein können durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$, worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl-und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

- für Heteroaryl stehen, die bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder eine Gruppe der Formel $-NR^5NR^6$ substituiert sein können, worin

$R^5$ und $R^6$ die oben angegebene Bedeutung haben,
oder
-für Aryl stehen, die bis zu 5-fach gleich oder verschieden substituiert sein können durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoy, Dialkylcarbamoyl oder durch durch eine Gruppe der Formel $-NR^5R^6$,
worin
$R^5$ und $R^6$ die oben angegebene Bedeutung haben,
dadurch gekennzeichnet, daß man Pyrrole der allgemeinen Formel

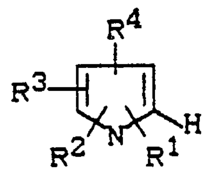

in welcher
$R^1$, $R^2$, $R^3$ und $R^4$ die oben angegebene Bedeutung haben,
in inerten Lösemitteln in Anwesenheit von Hilfsstoffen mit N,N-Dialkylaminoacrolein der Formel

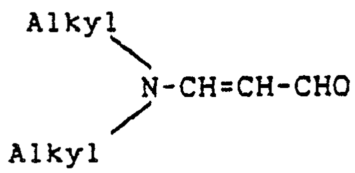

wobei
Alkyl für einen geradkettigen oder verzweigten Kohlenwasserstoffrest mit 1 bis 6 Kohlenstoffatomen steht, umsetzt.

10. Verfahren nach Anspruch 9, dadurch gekennzeichnet, daß es im Temperaturbereich von -20 °C bis +150 °C durchgeführt wird.